# EUROPEAN PATENT SPECIFICATION

(11) **EP 1 299 055 B1**
(45) Date of publication and mention of the grant of the patent: **15.10.2008**
(21) Application number: 01902949.5
(22) Date of filing: 07.02.2001
(51) Int. Cl.: A61F 5/00

(54) **CONTROLLED HEARTBURN AND REFLUX DISEASE TREATMENT APPARATUS**
GEREGELTE VORRICHTUNG ZUR BEHANDLUNG VON SODBRENNEN UND SAUREN AUFSTOSSEN
APPAREIL COMMANDE DE TRAITEMENT DE LA MALADIE DE REFLUX ET D'AIGREURS

(30) Priority: 10.02.2000 US 501235; 10.02.2000 US 501571
(43) Date of publication of application: 09.04.2003
(73) Proprietor: Obtech Medical AG, 6341 Baar (CH)
(72) Inventor: FORSELL, Peter, CH-6313 Menzingen (CH); DATTLER, Heinrich, CH-6315 Oberägeri (CH)
(74) Representative: Mercer, Christopher Paul
(86) International application number: PCT/SE2001/000229
(87) International publication number: WO 2001/045485

(56) References cited:
- WO-A1-00/09048
- WO-A1-00/15158
- WO-A1-01/12078
- US-A- 4 271 827
- US-A- 5 042 084
- US-A- 5 938 669

## Description

The present invention relates to a heartburn and reflux disease treatment apparatus, comprising a restriction device implantable in a patient, who suffers from heartburn and reflux disease, for engaging the stomach close to the cardia or engaging the esophagus, to form a restricted food passageway in the stomach or esophagus. The term "patient" includes an animal or a human being*.* The document US-A-5938669 represents the closest prior art.

Heartburn and reflux disease is a widespread medical problem. This is often due to hiatal hernia, i.e. a portion of the stomach immediately below the gastric fundus slides upwardly through the esophageal hiatus. In consequence, stomach acids and foods are regurgitated into the esophagus.

In the late 1970s a prior art restriction device called Angelchik, according to U.S. Patent No. 3,875,928, was used to operatively treat heartburn and reflux disease. However, the Angelchik restriction device had a major disadvantage in that it was not possible to adjust the size of the restriction opening after the operation. A further disadvantage was that the restriction device did not satisfactorily protect the esophagus and the surrounding area against injuries due to poor shape of the restriction device. Therefore, operations using the Angelchik restriction device are no longer practised.

An operation technique, semi-fundoduplicatio, is currently in use for treating heartburn and reflux disease. A most common operation is Nissen semi-fundoduplicatio, in which one takes the fundus of the stomach and makes a three quarter of a turn around the esophagus and suture between the stomach and esophagus. Although this operation works fairly well it has three main disadvantages. Firstly, most patient's treated in accordance to "ad modum Nissen" lose their ability to belch. Secondly, many of these patient's get dysphagia, i.e. difficulties to swallow after the operation. Thirdly, it is not possible to adjust the food passageway in the esophagus or stomach in any way after the operation. Characteristic for these patient's is the variation of their problems over the day. For example, many patient's have difficulties during the night when they lie down because of stomach acid leaking up into the esophagus.

It is an object of the present invention to provide a new convenient heartburn and reflux disease treatment apparatus, the performance of which may be affected by the patient at any time after operation, in particular when various needs arise over the day, so that the patient always is satisfied

This object is achieved by the features of claim 1.

As a result, the advantage is achieved that the restriction device can be non-invasively operated, when the restriction device has to be adjusted. Furthermore, the apparatus of the invention provides a simple and effective control of the energy supplied to implanted components of the apparatus which ensures an extended and reliable functionality of the apparatus, possibly for the rest of the patient's life and at least many years.

The control device may also control the restriction device. The control device may comprise an internal control unit, preferably including a microprocessor, implantable in the patient for controlling the restriction device. The control device may further comprise an external control unit outside the patient's body, wherein the internal control unit is programmable by the external control unit, for example for controlling the restriction device over time. Alternatively, the internal control unit may control the restriction device over time in accordance with an activity schedule program, which may be adapted to the patient's needs.

A great advantage is that the patient is enabled to adjust the restriction of the food passageway by using the control device whenever he likes during the day. This advantage should not be underestimated, because in case the patient would need to vomit it would be very difficult for him to do so if he were unable to immediately enlarge the food passageway.

Conveniently, the external control unit may load the internal control unit with data in accordance with a loading mode only authorized for a doctor. For specialized controls of the restriction device, the external control unit may control the internal control unit in accordance with a doctor mode only authorized for the doctor. For simple controls of the restriction device, the external control unit may control the internal control unit in accordance with a patient mode permitted for the patient. Thus, by using the external control unit in accordance with different modes it is possible to have certains functions of the restriction device controlled by the patient and other more advanced functions controlled by the doctor, which enables a flexible post-operation treatment of the patient.

The control device may be adapted to control the source of energy to release energy, for instance to intermittently release energy in the form of a train of energy pulses, for direct use in connection with the operation of the restriction device. In accordance with a suitable embodiment the control device controls the source of energy to release electric energy, and the apparatus further comprises an implantable capacitor for producing the train of energy pulses from the released energy. In this case the term "*direct*" is used to mean, on one hand, that the released energy is used while it is being released by the control device, on the other hand, that the released energy may be somewhat delayed, in the order of seconds, by for instance an energy stabilizer before being used in connection with the operation of the restriction device. The restriction device may be operable in non-manual, a non-magnetic or non-mechanical manner by use of the released energy.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

Generally, the apparatus further comprises an operation device implantable in the patient for operating the restriction device, wherein the control device controls the operation device to operate the restriction device. The control device may directly power the operation device with energy released from the source of energy and/or power other implantable energy consuming components of the apparatus. In this case the term "*directly*" is used to mean, on one hand, that the operation device is powered with released energy while the latter is being released by the control device, on the other hand, that the released energy may be somewhat delayed, in the order of seconds, by for instance an energy stabilizer before powering the operation device. The advantage of directly using energy as it is released is that the apparatus can be of a very simple design and the few components involved makes the apparatus extremely reliable.

The restriction device may be non-inflatable, i.e. with no hydraulic fluid involved for the adjustments of the restriction device. This eliminates problems with fluid leaking from the restriction device.

The operation device may comprise hydraulic means and at least one valve for controlling a fluid flow in the hydraulic means. The control device may suitably comprise a wireless remote control for controlling the valve. The restriction device may comprise hydraulic means and the operation device may comprise a reservoir forming a fluid chamber with a variable volume connected to the hydraulic means. The operation device may distribute fluid from the chamber to the hydraulic means by reduction of the volume of the chamber and withdraw fluid from the hydraulic means to the chamber by expansion of the volume of the chamber.

In accordance with a first main aspect of the invention, the source of energy is external to the patient's body and the control device controls the source of energy to release wireless energy. The external source of energy may be of any conceivable kind, such as a nuclear source of energy or a chemical source of energy.

An energy storage device, preferably an electric accumulator, may be implantable in the patient for storing the wireless energy released from the external source of energy. The electric accumulator may comprise at least one capacitor or at least one rechargeable battery, or a combination of at least one capacitor and at least one rechargeable battery. Alternatively, a battery may be implantable in the patient for supplying electric energy to implanted electric energy consuming components of the apparatus, in addition to the supply of wireless energy. Where the control device comprises an implantable control unit the electronic circuit thereof and the restriction device may be directly powered with transformed wireless energy, or energy from either the implantable energy storage device or battery.

In accordance with a second main aspect of the invention, the wireless energy is directly used for operation of the restriction device, i.e. the restriction device is operated as the wireless energy is released from the external source of energy by the control device. In this case the term "*directly*" is used to mean, on one hand, that the restriction device is promptly operated by using the released energy whithout first storing the latter, on the other hand, that the released energy may be somewhat delayed, in the order of seconds, by for instance an energy stabilizer before being used for the operation of the restriction device. As a result, a very simple control of the restriction device is achieved and there are only a few implanted components of the apparatus. For example, there is no implanted source of energy, such as a battery, nor any implanted complicated signal control system. This gives the advantage that the apparatus will be extremely reliable.

Generally, the control device controls and directly or indirectly powers the operation device with wireless energy released from the source of energy and/or powers other implanted energy consuming components of the apparatus.

In a first particular embodiment in accordance with the first and second main aspects of the invention, the operation device comprises a motor, preferably an electric motor which may have electrically conductive parts made of plastics. The motor may include a rotary motor, wherein the control device is adapted to control the rotary motor to rotate a desired number of revolutions. Alternatively, the motor may include a linear motor, or a hydraulic or pneumatic fluid motor, wherein the control device is adapted to control the fluid flow through the fluid motor. Motors currently available on the market are getting smaller and smaller. Furthermore, there is a great variety of control methods and miniaturized control equipment available. For example, a number of revolutions of a rotary motor may be analyzed by a Hall-element just a few mm in size.

In a second particular embodiment in accordance with the first and second main aspects of the invention, the control device is adapted to shift polarity of the released energy to reverse the operation device. The operation device may suitably comprise an electric motor and the released energy may comprise electric energy.

In a third particular embodiment in accordance with the first and second main aspects of the invention, the restriction device is operable to perform a reversible function and there is a reversing device implantable in the patient for reversing the function performed by the restriction device. Such a reversing function preferably involves enlarging and restricting the food passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

In accordance with the third particular embodiment, the operation device preferably comprises a motor, wherein the reversing device reverses the motor.

In a fourth particular embodiment in accordance with the first and second main aspects of the invention, the restriction device comprises hydraulic means, for example including an expansible/contractible cavity for fluid. Preferably, the operation device is adapted to conduct hydraulic fluid in the hydraulic means, and comprises a motor, a valveless fluid conduit connected to the hydraulic means of the restriction device, and a reservoir for fluid, wherein the reservoir forms part of the conduit. The operation device suitably comprises a pump operated by the motor. All of the hydraulic components involved are preferably deviod of any non-return valve. This is of great advantage, because with valves involved there is always a risk of malfunction due to inproperly working valves, especially when long time periods passes between valve operations. The reservoir may form a fluid chamber with a variable volume, and the pump may distribute fluid from the chamber to the hydraulic means of the restriction device by reduction of the volume of the chamber and withdraw fluid from the hydraulic means to the chamber by expansion of the volume of the chamber.

In accordance with a third main aspect of the invention, the source of energy is implantable in the patient. Thus, when the source of energy is implanted in a patient the control device controls it from outside the patient's body to release energy. This solution is advantageous for embodiments of the apparatus that have a relatively high consumption of energy which cannot be satisfied by direct supply of wireless energy.

The implantable source of energy may comprise an accumulator, preferably an electric source of energy, such as a battery having a lifetime of at least 10 years.

In accordance with a fourth main aspect of the invention, the apparatus comprises a switch implanted in the patient for directly or indirectly switching the operation of the restriction device and an internal source of energy, such as a battery, implanted in the patient for supplying energy for the operation of the restriction device, wherein the switch directly or indirectly affects the supply of energy from the internal source of energy. This solution is advantageous for embodiments of the apparatus that have a relatively high energy consumption which cannot be met by direct supply of wireless energy.

In a first particular embodiment in accordance with the fourth main aspect of the invention, the switch switches between an off mode, in which the internal source of energy is not in use, and an on mode, in which the internal source of energy supplies energy for the operation of the restriction device. In this case, the switch is conveniently operated by the wireless energy released from the external source of energy to switch between the on and off modes. The control device, preferably comprising a wireless remote control, may control the external source of energy to release the wireless energy. The advantage of this embodiment is that the lifetime of the implanted source of energy, such as a battery, can be significantly prolonged, since the implanted source of energy does not supply energy when the switch is in its off mode.

In a second particular embodiment in accordance with the fourth main aspect of the invention, the control device comprises a wireless remote control for controlling the internal source of energy. In this case, the switch is operable by the wireless energy from the external source of energy to switch between an off mode, in which the internal source of energy and remote control are not in use, and a standby mode, in which the remote control is permitted to control the internal source of energy to supply energy for the operation of the restriction device.

In a third particular embodiment in accordance with the fourth main aspect of the invention, the apparatus further comprises an energy transforming device implanted in the patient for transforming the wireless energy into storable energy, wherein the internal source of energy is capable of storing the storable energy. The internal source of energy preferably comprises an electric accumulator, at least one capacitor or at least one rechargeable battery, or a combination of at least one capacitor and at least one rechargeable battery. In this case, the switch switches from an off mode, in which the internal source of energy is not in use, to an on mode, in which the internal source of energy supplies energy for the operation of the restriction device.

The control device, preferably comprising a wireless remote control, may control the switch to switch between the on and off modes.

Alternatively, in this third particular embodiment an energy storage device may be implanted in the patient for storing the storable energy instead of the internal source of energy, wherein the switch is operable by energy from the implanted energy storage device to switch between an off mode, in which the internal source of energy is not in use, and an on mode, in which the internal source of energy supplies energy for the operation of the restriction device. In this case, the control device (the wireless remote control) controls the energy storage device to operate the switch.

The internal source of energy preferably comprises an electric source of energy, such as an accumulator or a battery having a lifetime of at least 10 years. However, other kinds of sources are also conceivable, such as a nuclear source of energy or a chemical source of energy.

The above first, second, third and fourth particular embodiments described in connection with the first and second main aspects of the invention are also applicable in accordance with the third main aspect of the invention, i.e. where the source of energy is implantable, and in accordance with the fourth main aspect of the invention, i.e. where the apparatus comprises an implantable switch.

All of the above embodiments may be combined with at least one implantable sensor for sensing at least one physical parameter of the patient, wherein the control device may control the restriction device in response to signals from the sensor. For example, the sensor may comprise a pressure sensor for directly or indirectly sensing the pressure in the food passageway. The expression "*indirectly sensing the pressure in the food passageway*" should be understood to encompass the cases where the sensor senses the pressure against the restriction device or human tissue of the patient. Where the control device comprises an internal control unit to be implanted in the patient, the internal control unit may suitably directly control the restriction device in response to signals from the sensor. In response to signals from the sensor, for example pressure, the patient's position or any other important physical parameter, the internal control unit may send information thereon to outside the patient's body. The control unit may also automatically control the restriction device in response to signals from the sensor. For example, the control unit may control the restriction device to further restrict the food passageway in the stomach in response to the sensor sensing that the patient is lying, or enlarge the food passageway in response to the sensor sensing an abnormally high pressure against the restriction device.

Where the control device comprises an external control unit outside the patient's body, the external control unit may, suitably directly, control the restriction device in response to signals from the sensor. The external control unit may store information on the physical parameter sensed by the sensor and may be manually operated to control the restriction device based on the stored information. In addition, there may be at least one implantable sender for sending information on the physical parameter sensed by the sensor.

An external data communicator may be provided outside the patient's body and an internal data communicator to be implanted in the patient may be provided for communicating with the external data communicator. The internal data communicator may feed data related to the patient, or related to the restriction device, back to the external data communicator. Alternatively or in combination, the external data communicator may feed data to the internal data communicator. The internal data communicator may suitably feed data related to at least one physical signal of the patient.

Generally, the apparatus of the invention may comprise a switch implantable in the patient for directly or indirectly switching the energy released from the source of energy. For example, the restriction device may be operable to open and close the food passageway or may steplessly control the cross-sectional area of the food passageway. A pressure sensor may be provided for directly or indirectly sensing the pressure in the food passageway. The control device may control the restriction device in response to signals from the pressure sensor.

The apparatus may comprise an implantable energy transforming device, wherein the control device releases electric energy and the energy transforming device transforms the electric energy into kinetic energy for, preferably direct, operation of the restriction device. Suitably, an implantable stabilizer, such as a capacitor or a rechargeable accumulator, or the like, may be provided for stabilizing the electric energy released by the control device. In addition, the control device may control the source of energy to release energy for a determined time period or in a determined number of energy pulses. Finally, the restriction device may be non-inflatable.

All of the above embodiments are preferably remote controlled. Thus, the control device advantageously comprises a wireless remote control transmitting at least one wireless control signal for controlling the restriction device. With such a remote control it will be possible to adapt the function of the apparatus to the patient's need in a daily basis, which is beneficial with respect to the treatment of the patient.

The wireless remote control may be capable of obtaining information on the condition of the restriction device and of controlling the restriction device in response to the information. Also, The remote control may be capable of sending information related to the restriction device from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal reciever or transceiver and at least one internal signal transmitter or transceiver implantable in the patient.

The remote control may transmit a carrier signal for carrying the control signal, wherein the carrier signal is frequency, amplitude or frequency and amplitude modulated and is digital, analog or digital and analog. Also the control signal used with the carrier signal may be frequency, amplitude or frequency and amplitude modulated.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analog, and may comprise an electric or magnetic field. Suitably, the wireless remote control may transmit an electromagnetic carrier wave signal for carrying the digital or analog control signal. For example, use of an analog carrier wave signal carrying a digital control signal would give safe communication. The control signal may be transmitted in pulses by the wireless remote control.

In all of the above solutions, the control device advantageously releases energy from the source of energy in a non-invasive, magnetic, non-magnetic, mechanical or non-mechanical manner.

The control device may release magnetic, electromagnetic, kinetic, sonic or thermal energy, or non-magnetic, non-sonic, non-thermal, non-electromagnetic or non-kinetic energy.

The control device may be activated in a manual or non-manual manner to control the source of energy to release energy.

The operation device may be powered by magnetic energy, non-magnetic energy, electromagnetic energy, non-electromagnetic energy, kinetic energy, non-kinetic energy, thermal energy or non-thermal energy. However, preferably the operation device comprises an electrical operation device.

Typically the apparatus of the invention comprises an adjustment device for adjusting the restriction device to change the restriction of the food passageway. The adjustment device may be adapted to mechanically adjust the restriction device. Alternatively, the adjustment device may be adapted to hydraulically adjust the restriction device by using hydraulic means which is devoid of hydraulic fluid of the kind having a viscosity that substantially increases when exposed to heat or a magnetic field, i.e. the hydraulic fluid would not become more viscous when exposed to heat or influenced by magnetic forces.

The above-presented embodiments of the invention may be modified in accordance with the following suggestions. The released energy may comprise electric energy and an implantable capacitor having a capacity less than 0,1 µF may be provided for producing the above-mentioned train of energy pulses.

An implantable motor or pump may be provided for operating the restriction device, wherein the control device is adapted to control the source of energy to directly power the motor or pump with the released energy. Specifically, the control device may be adapted to release wireless energy in the form of a magnetic field or electromagnetic waves (excluding radio waves) for direct power of the motor or pump, as the wireless energy is being released. Where a pump is used it preferably is not a plunger type of pump.

Generally, the wireless energy comprises a signal.

The apparatus may further comprise implantable energy transforming device for transforming wireless energy directly or indirectly into energy different than the wireless energy, for operation of the restriction device. For example, the motor or pump may be powered by the transformed energy.

The energy transforming device may transform the wireless energy in the form of sound waves, preferably directly, into electric energy for operation of the restriction device. The energy transforming device may comprise a capacitor adapted to produce electric pulses from the transformed electric energy.

The motor mentioned in the present specification may also be directly powered with wirelessly transmitted electromagnetic or magnetic energy in the form of signals, as the energy is transmitted. Furthermore, all the various functions of the motor and associated components described in the present specification may be used where applicable.

Generally, the restriction device advantageously is embedded in a soft or gel-like material, such as a silicone material having hardness less than 20 Shore.

Of course, the restriction device preferably is adjustable in a non-manual manner.

All the above described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

The invention is described in more detail in the following with reference to the accompanying drawings, in which
FIGURES 1 to 6 are schematic block diagrams illustrating six embodiments, respectively, of the invention, in which wireless energy is released from an external source of energy;
FIGURES 7 to 10 are schematic block diagrams illustrating four embodiments, respectively, of the invention, in which energy is released from an implanted source of energy;
FIGURES 11 to 15 are schematic block diagrams illustrating five embodiments, respectively, of the invention, in which a switch is implanted in the patient for directly or indirectly switching the operation of the restriction device;
FIGURE 16 is a schematic block diagram illustrating conceivable combinations of implantable components for achieving various communication options;
FIGURE 17 illustrates the apparatus in accordance with the invention implanted in a patient;
FIGURE 18 is a block diagram illustrating remote control components of an embodiment of the invention; and
FIGURE 19 is a schematic view of exemplary circuitry used for the components of the block diagram of FIGURE 18.
Referring to the drawing figures, like reference numerals designate identical or corresponding elements throughout the several figures.

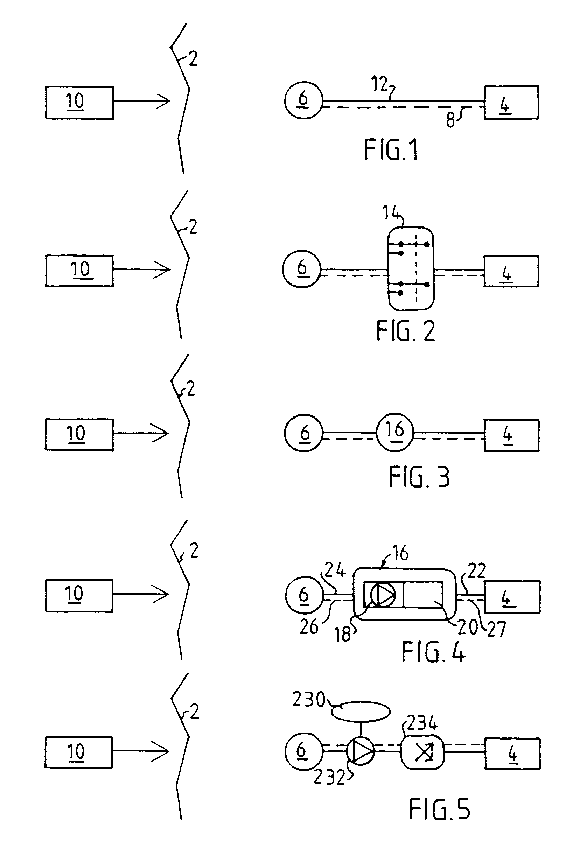

FIGURE 1 schematically shows an embodiment of the heartburn and reflux disease apparatus of the invention having some parts implanted in a patient and other parts located outside the patient's body. Thus, in FIGURE 1 all parts placed to the right of the patient's skin 2 are implanted and all parts placed to the left of the skin 2 are located outside the patient's body. The apparatus of FIGURE 1 comprises an implanted operable restriction device 4, which engages the patient's stomach close to the cardia (or alternatively engages the esophagus) to form a restricted food passageway in the stomach. The restriction device 4 is capable of performing a reversible function, i.e. to enlarge and reduce the cross-sectional area of the food passageway, whereby the restriction device works as an artificial sphincter. An implanted control unit 6 controls the restriction device 4 via a control line 8 to form an adequate size of the cross-sectional area of the restricted food passageway. An external control unit 10 includes an external source of energy and a wireless remote control transmitting a control signal generated by the external source of energy. The control signal is received by a signal receiver incorporated in the implanted control unit 6, whereby the control unit 6 controls the implanted restriction device 4 in response to the control signal. The implanted control unit 6 also uses energy from the control signal for operating the restriction device 4 via a power supply line 12.

FIGURE 2 shows an embodiment of the invention identical to that of FIGURE 1, except that a reversing device in the form of a switch 14 operable by energy also is implanted in the patient for reversing the restriction device 4. The control unit 6 uses the switch 14 to reverse the function performed by the restriction device 4. More precisely, the external control unit 10 releases energy carried by a wireless signal and the implanted control unit 6 transforms the wireless energy into a current for operating the switch 14. When the control unit 6 shifts the polarity of the current the switch 14 reverses the function performed by the restriction device 4.

FIGURE 3 shows an embodiment of the invention identical to that of FIGURE 1, except that an operation device in the form of a motor 16 also is implanted in the patient. The implanted control unit 6 powers the motor 16 with wireless energy released from the external source of energy of the external control unit 10. The implanted control unit 6 controls the operation of the motor 16 in response to a control signal from the remote control of the external control unit 10.

FIGURE 4 shows an embodiment of the invention identical to that of FIGURE 1, except that an assembly 16 including a motor/pump unit 18 and a fluid reservoir 20 also is implanted in the patient. In this case the restriction device 4 is hydraulically operated, i.e. hydraulic fluid is pumped by the motor/pump unit 18 from the reservoir 20 through a conduit 22 to the restriction device 4 to reduce the cross-sectional area of the food passageway, and hydraulic fluid is pumped by the motor/pump unit 18 back from the restriction device 4 to the reservoir 20 to enlarge the cross-sectional area. The external control unit 10 releases energy carried by a wireless signal and the implanted control unit 6 transforms the wireless energy into a current, for example a current, for powering the motor/pump unit 18 via an electric power supply line 24. The implanted control unit 6 controls the motor/pump unit 16 and the restriction device 4 via control lines 26 and 27.

FIGURE 5 shows an embodiment of the invention comprising the restriction device 4, hydraulically operated, and the implanted control unit 6, and further comprising a hydraulic fluid reservoir 230, a motor/pump unit 232 and a reversing device in the form of a hydraulic valve shifting device 234, all of which are implanted in the patient. The motor of the motor/pump unit 232 is an electric motor.

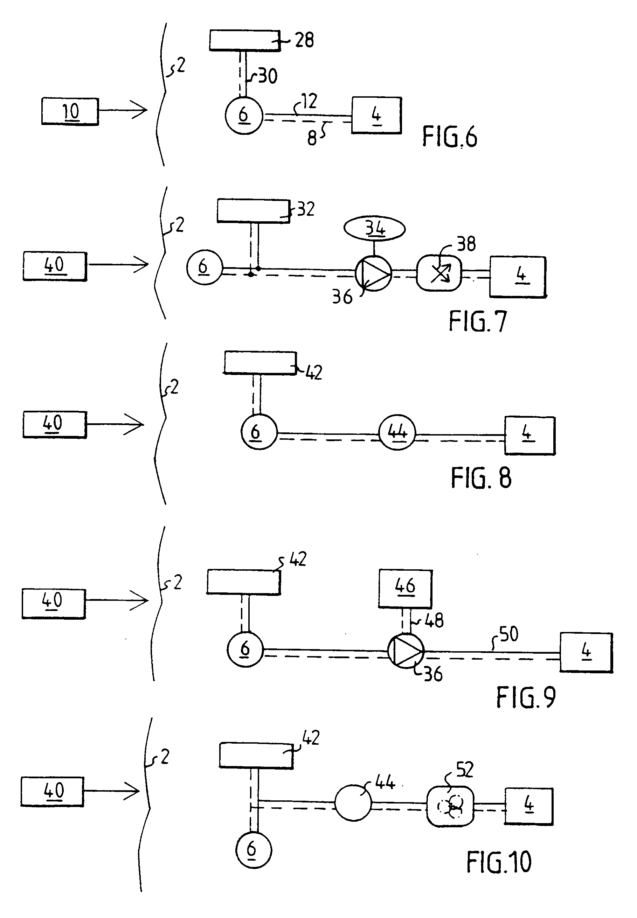

FIGURE 6 shows an embodiment of the invention identical to that of FIGURE 1, except that an accumulator 28 also is implanted in the patient. The control unit 6 stores energy received from the external control unit 10 in the accumulator 28. In response to a control signal from the external control unit 10 the implanted control unit 6 releases energy from the accumulator 28 via a power line 30 for the operation of the restriction device 4.

FIGURE 7 shows an embodiment of the invention comprising the restriction device 4, hydraulically operated, and the implanted control unit 6, and further comprising a source of energy in the form of a battery 32, a hydraulic fluid reservoir 34, a motor/pump unit 36 and a reversing device in the form of a hydraulic valve shifting device 38, all of which are implanted in the patient. The motor of the motor/pump unit 36 is an electric motor. An external control unit 40 includes a wireless remote control transmitting a control signal which is received by the signal receiver incorporated in the implanted control unit 6.

In response to a control signal from the external control unit 40 the implanted control unit 6 powers the motor/pump unit 36 with energy from the battery 32, whereby the motor/pump unit 36 distributes hydraulic fluid between the reservoir 34 and the restriction device 4. The control unit 6 controls the shifting device 38 to shift the hydraulic fluid flow direction between one direction in which the fluid is pumped by the motor/pump unit 36 from the reservoir 34 to the restriction device 4 to reduce the cross-sectional area of the food passageway, and another opposite direction in which the fluid is pumped by the motor/pump unit 36 back from the restriction device 4 to the reservoir 34 to enlarge the cross-sectional area.

FIGURE 8 shows an embodiment of the invention identical to that of FIGURE 6, except that a battery 42 is substituted for the accumulator 28, the external control unit 40 of the embodiment of FIGURE 5 is substituted for the external control unit 10 and an electric motor 44 is implanted in the patient for operating the restriction device 4. In response to a control signal from the external control unit 40 the implanted control unit 6 powers the motor 44 with energy from the battery 42, whereby the motor 44 operates the restriction device 4.

FIGURE 9 shows an embodiment of the invention identical to that of FIGURE 8, except that the motor/pump unit 36 of the embodiment of FIGURE 7 is substituted for the motor 44 and a fluid reservoir 46 also is implanted in the patient. The reservoir 46 is via fluid conduits 48 and 50 connected to the motor/pump unit 36 and restriction device 4, which in this case is hydraulically operated. In response to a control signal from the external control unit 40, the implanted control unit 6 powers the electric motor of the motor/pump unit 36 with energy from the battery 42, whereby the motor/pump unit 36 distributes hydraulic fluid between the fluid reservoir 46 and the restriction device 4.

FIGURE 10 shows an embodiment of the invention identical to that of FIGURE 8, except that a mechanical reversing device in the form of a gearbox 52 also is implanted in the patient. The implanted control unit 6 controls the gearbox 52 to reverse the function performed by the restriction device 4 (mechanically operated).

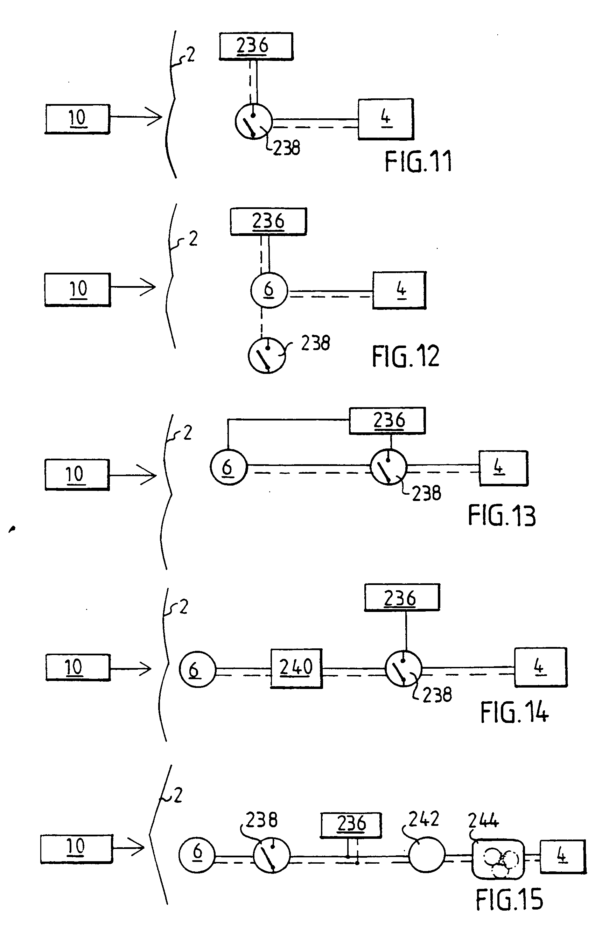

FIGURE 11 shows an embodiment of the invention comprising the restriction device 4, the external control unit 10, an implanted source of energy 236 and an implanted switch 238. The switch 238 is operated by wireless energy released from the external source of energy of the external control unit 6 to switch between an off mode, in which the implanted source of energy 236 is not in use, and an on mode, in which the implanted source of energy 236 supplies energy for the operation of the restriction device 4.

FIGURE 12 shows an embodiment of the invention identical to that of FIGURE 11, except that also the control unit 6 is implanted, in order to receive a control signal from the wireless remote control of the external control unit 10. The switch 238 is operated by the wireless energy from the external source of energy 10 to switch between an off mode, in which the implanted source of energy 236 and the wireless remote control of the external control unit 10 are not in use, i.e. the control unit 6 is not capable of receiving the control signal, and a standby mode, in which the wireless remote control is permitted to control the internal source of energy 236, via the implanted control unit 6, to supply energy for the operation of the restriction device 4.

FIGURE 13 shows an embodiment of the invention identical to that of FIGURE 12, except that an energy transforming device for transforming the wireless energy into storable energy is incorporated in the implanted control unit 6 and that the implanted source of energy 236 is of a type that is capable of storing the storable energy. In this case, in response to a control signal from the external control unit 10, the implanted control unit 6 controls the switch 238 to switch from an off mode, in which the implanted source of energy 236 is not in use, to an on mode, in which the source of energy 36 supplies energy for the operation of the restriction device 4.

FIGURE 14 shows an embodiment of the invention identical to that of FIGURE 13, except that an energy storage device 240 also is implanted in the patient for storing the storable energy transformed from the wireless energy by the transforming device of the control unit 6. In this case, the implanted ontrol unit 6 controls the energy storage device 240 to operate the switch 238 to switch between an off mode, in which the implanted source of energy 236 is not in use, and an on mode, in which the implanted source of energy 236 supplies energy for the operation of the restriction device 4.

FIGURE 15 shows an embodiment of the invention identical to that of FIGURE 13, except that a motor 242 and a mechanical reversing device in the form of a gearbox 244 also are implanted in the patient. The implanted control unit 6 controls the gearbox 244 to reverse the function performed by the restriction device 4 (mechanically operated), i.e. enlarging and restricting the food passageway.

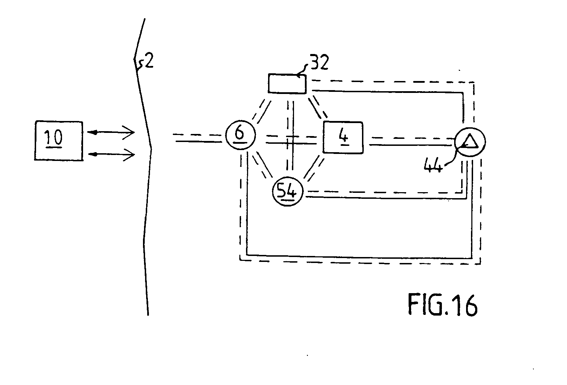

FIGURE 16 schematically shows conceivable combinations of implanted components of the apparatus for achieving various communication possibilities. Basically, there are the implanted restriction device 4, the implanted control unit 6 and the external control unit 10 including the external source of energy and the wireless remote control. As already described above the remote control transmits a control signal generated by the external source of energy, and the control signal is received by a signal receiver incorporated in the implanted control unit 6, whereby the control unit 6 controls the implanted restriction device 4 in response to the control signal.

A sensor 54 may be implanted in the patient for sensing a physical parameter of the patient, such as the pressure in the food passageway. The control unit 6, or alternatively the external control unit 10, may control the restriction device 4 in response to signals from the sensor 54. A transceiver may be combined with the sensor 54 for sending information on the sensed physical parameter to the external control unit 10. The wireless remote control of the external control unit 10 may comprise a signal transmitter or transceiver and the implanted control unit 6 may comprise a signal receiver or transceiver. Alternatively, the wireless remote control of the external control unit 10 may comprise a signal receiver or transceiver and the implanted control unit 6 may comprise a signal transmitter or transceiver. The above transceivers, transmitters and receivers may be used for sending information or data related to the restriction device from inside the patient's body to the outside thereof.

The motor 44 may be implanted for operating the restriction device 4 and also the battery 32 may be implanted for powering the motor 44. The battery 32 may be equipped with a transceiver for sending information on the charge condition of the battery.

Those skilled in the art will realize that the above various embodiments according to FIGURES 1-15 could be combined in many different ways. For example, the energy operated switch 14 could be incorporated in any of the embodiments of FIGURES 4,6,8-10. The hydraulic shifting device 38 could be incorporated in any of the embodiments of FIGURES 4 and 9. The gearbox 52 could be incorporated in any of the embodiments of FIGURES 1,6 and 8.

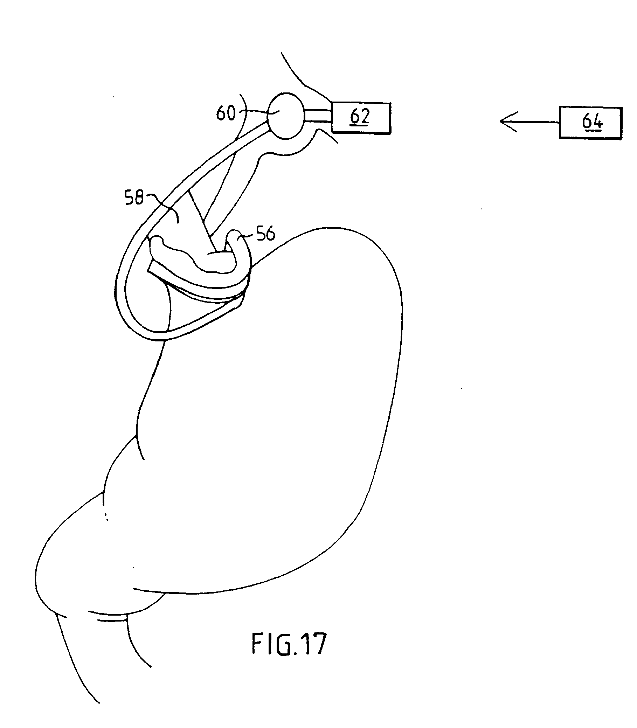

FIGURE 17 illustrates how any of the above-described embodiments of the heartburn and reflux disease treatment apparatus of the invention may be implanted in a patient. Thus, an assembly of the apparatus implanted in the patient comprises a restriction device 56 engaging the esophagus 58 close to the cardia, and an operation device 60 for operating the restriction device 56 and an internal control unit 62, which includes a signal receiver, for controlling the operation device 60. An external control unit 64 includes a signal transmitter for transmitting a control signal to the signal receiver of the implanted control unit 62. The implanted control unit 62 is capable of transforming signal energy from the control signal into electric energy for powering the operation device 60 and for energizing energy consuming implanted components of the apparatus.

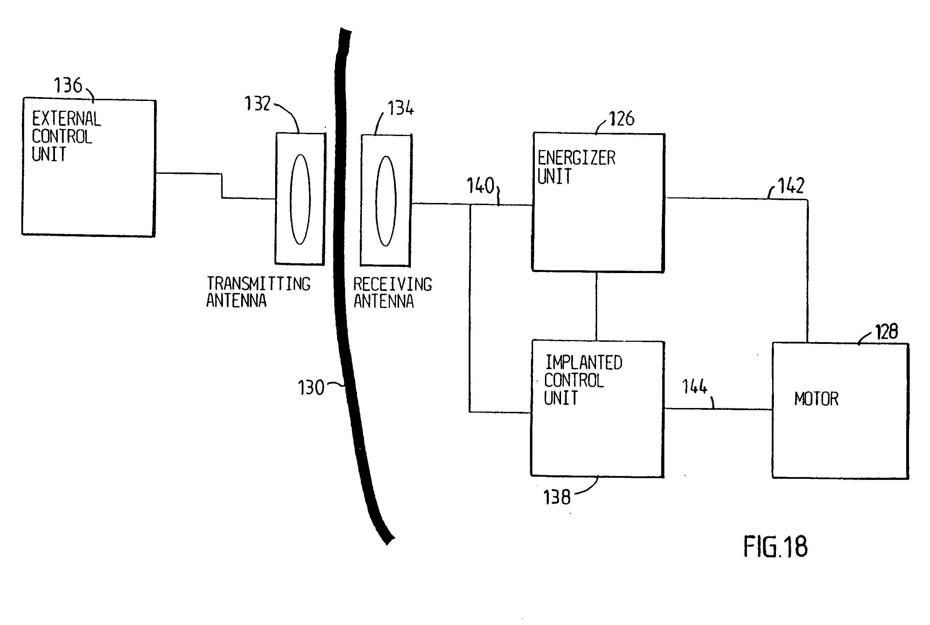

FIGURE 18 shows the basic parts of a wireless remote control of the apparatus of the invention including an electric motor 128 for operating a restriction member, for example of the type illustrated in FIGURE 17. In this case, the remote control is based on the transmission of electromagnetic wave signals, often of high frequencies in the order of 100 kHz - 1 gHz, through the skin 130 of the patient. In FIGURE 18, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same size as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to either contract or enlarge the restriction device. The microprocessor starts a command by applying a high frequency signal on the antenna 132. After a short time, when the signal has energized the implanted parts of the control system, commands are sent to contract or enlarge the restriction device in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new contract or enlarge step is desired the Count byte is increased by one to allow the implanted control unit 138 to decode and understand that another step is demanded by the external control unit 136. If any part of the digital packet is erroneous, its content is simply ignored.

Through a line 140, an implanted energizer unit 126 draws energy from the high frequency electromagnetic wave signals received by the receiving antenna 134. The energizer unit 126 stores the energy in a power supply, such as a large capacitor, powers the control unit 138 and powers the electric motor 128 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energizer unit 126 has sufficient energy stored, sends a signal via a signal line 144 to the motor 128 to either contract or enlarge the restriction device depending on the received command code.

Alternatively, the energy stored in the power supply of the energizer unit may only be used for powering a switch, and the energy for powering the motor 128 may be obtained from another implanted power source of relatively high capacity, for example a battery. In this case the switch is adapted to connect said battery to the control unit 138 in an on mode when said switch is powered by said power supply and to keep said battery disconnected from the control unit in a standby mode when said switch is unpowered.

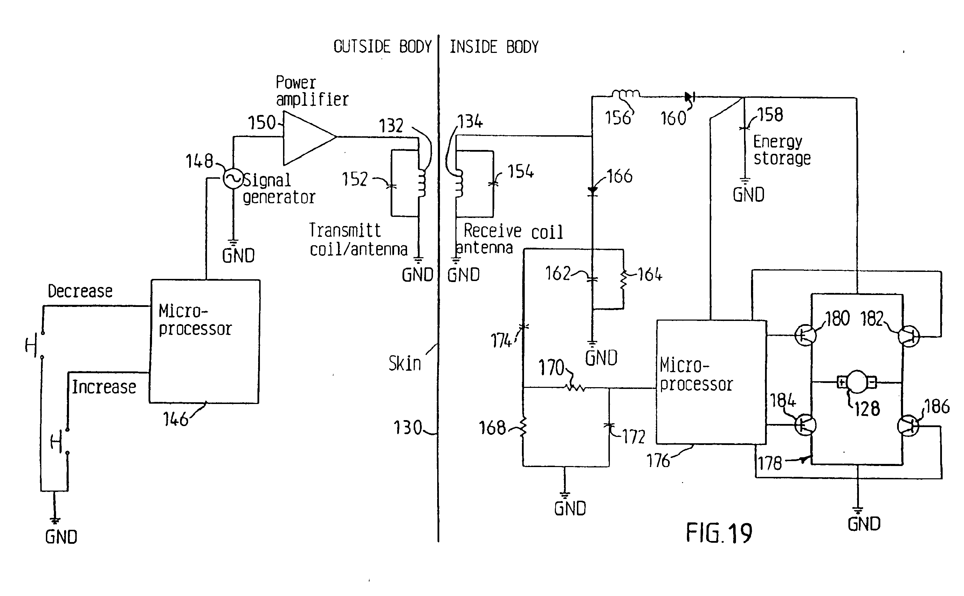

With reference to FIGURE 19, the remote control schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 128 via an H-bridge 178 comprising transistors 180,182,184 and 186. The motor 128 can be driven in two opposite directions by the H-bridge 178.

The microprocessor 176 also monitors the amount of stored energy in the storage capacitor 158. Before sending signals to activate the motor 128, the microprocessor 176 checks whether the energy stored in the storage capacitor 158 is enough. If the stored energy is not enough to perform the requested operation, the microprocessor 176 waits for the received signals to charge the storage capacitor 158 before activating the motor 128.

## Claims

1. A heartburn and reflux disease treatment apparatus, comprising a restriction device implantable in a patient, who suffers from heartburn and reflux disease, for engaging the stomach close to the cardia or engaging the esophagus, to form a restricted food passageway in the stomach or esophagus, the restriction device being operable to change the restriction of the restricted food passageway, wherein a source of energy is provided, and a control device operable from outside the patient's body is provided for controlling the source of energy to release energy for use in connection with the operation of the restriction device, when the restriction device is implanted, wherein the control device comprises a programmable internal control unit implantable in the patient for controlling the restriction device and an external control unit intended to be outside the patient's body, the internal control unit being programmable by the external control unit, and wherein the external control unit is adapted to load the internal control unit with data, or to control the internal control unit, in accordance with a doctor mode only authorized for a doctor **characterised in that** the external control unit can be further used in accordance with a patient mode permitted for the patient wherein, in use, the patient mode allows the patient to access first functions of the internal control unit and the doctor mode allows the doctor to access second functions of the internal control unit.

2. An apparatus according to claim 1, wherein the source of energy is intended to be external to the patient's body when the restriction device is implanted therein, and the control device is adapted to control the external source of energy to release wireless energy for use in connection with the operation of the restriction device.

3. An apparatus according to claim 1, wherein the internal control unit is programmable for controlling the restriction device over time.

4. An apparatus according to claim 3, wherein the internal control unit controls the restriction device over time in accordance with an activity schedule program.

5. An apparatus according to claim 3, wherein the internal control unit comprises a microprocessor.

6. An apparatus according to claim 1, wherein the source of energy is implantable in the patient.

7. An apparatus according to claim 6, wherein the implantable source of energy comprises at least one accumulator, at least one capacitor or at least one rechargeable battery, or a combination of at least one capacitor and at least one rechargeable battery.

8. An apparatus according to claim 7, wherein the implantable source of energy comprises an electric source of energy.

9. An apparatus according to claim 8, wherein the electric source of energy comprises an accumulator, or a battery having a lifetime of at least 10 years.

10. An apparatus according to claim 2, further comprising an energy storage device implantable in the patient for storing the wireless energy released from the external source of energy.

11. An apparatus according to claim 10, wherein the energy storage device comprises an accumulator.

12. An apparatus according to claim 11, wherein the accumulator comprises an electric accumulator.

13. An apparatus according to claim 12, wherein the electric accumulator comprises at least one capacitor or at least one rechargeable battery, or a combination of at least one capacitor and at least one rechargeable battery.

14. An apparatus according to claim 1 or 2, further comprising a battery implantable in the patient for supplying electric energy to implantable electric energy consuming components of the apparatus.

15. The apparatus according to claim 2, wherein the control device is adapted to control the external source of energy to release wireless energy for direct use in connection with the operation of the restriction device.

16. The apparatus according to claim 15, wherein the control device is adapted to control the external source of energy to intermittently release wireless energy in the form of a train of energy pulses for direct use in connection with the operation of the restriction device.

17. The apparatus according to claim 15 or 16, wherein the restriction device is operable in a non-magnetic, non-thermal or non-mechanical manner by use of said released wireless energy.

18. The apparatus according to claims 1 or claim 2, further comprising a switch implantable in the patient for directly or indirectly switching the operation of the restriction device.

19. The apparatus according to claim 18, further comprising an internal source of energy implantable in the patient for supplying energy for the operation of the restriction device, wherein the switch directly or indirectly affects the supply of energy from the internal source of energy.

20. The apparatus according to claim 19, wherein the switch switches between an "off" mode, in which the internal source of energy is not in use, and an "on" mode, in which the internal source of energy supplies energy for the operation of the restriction device.

21. The apparatus according to claim 2 and 20, wherein the switch is operable by the wireless energy released from the external source of energy.

22. The apparatus according to claim 21, wherein the control device controls the external source of energy to release the wireless energy.

23. The apparatus according to any of claims 1, 2 and 22, wherein the control device comprises a wireless remote control.

24. The apparatus according to claim 19, wherein the control device comprises a wireless remote control for controlling the internal source of energy.

25. The apparatus according to claim 24, wherein the switch is operable by the wireless energy from the external source of energy to switch between an "off" mode, in which the internal source of energy and remote control are not in use, and a "standby" mode, in which the remote control is permitted to control the internal source of energy to supply energy for the operation of the restriction device.

26. The apparatus according to claim 19, further comprising an energy transforming device implantable in the patient for transforming the wireless energy into storable energy and an energy storage device implantable in the patient for storing the storable energy.

27. The apparatus according to claim 26, wherein the switch is operable by energy from the implantable energy storage device to switch between an "off" mode, in which the internal source of energy is not in use, and an "on" mode, in which the internal source of energy supplies energy for the operation of the restriction device.

28. The apparatus according to claim 27, wherein the control device controls the energy storage device to operate the switch.

29. The apparatus according to claim 28, wherein the control device comprises a wireless remote control.

30. The apparatus according to claim 29, further comprising an energy transforming device implantable in the patient for transforming the wireless energy into storable energy, wherein the internal source of energy is capable of storing the storable energy.

31. The apparatus according to claim 30, wherein the switch switches from an "off" mode, in which the internal source of energy is not in use, to an "on" mode, in which the source of energy supplies energy for the operation of the restriction device.

32. The apparatus according to claim 31, wherein the control device controls the switch to switch between the "on" and "off" modes.

33. The apparatus according to claim 32, wherein the control device comprises a wireless remote control.

34. The apparatus according to claim 19, wherein the internal source of energy comprises an electric source of energy.

35. The apparatus according to claim 34, wherein the electric source of energy comprises at least one accumulator, at least one capacitor or at least one rechargeable battery, or a combination of at least one capacitor and at least one rechargeable battery.

36. The apparatus according to claim 34, wherein the electric source of energy comprises an accumulator or a battery having a lifetime of at least 10 years.

37. The apparatus according to claim 1, wherein the source of energy comprises a chemical source of energy.

38. An apparatus according to any one of claims 1, 2, 6 or 18, further comprising an operation device implantable in the patient for operating the restriction device.

39. An apparatus according to claim 2 or 15, further comprising an operation device implantable in the patient for operating the restriction device, wherein the wireless energy directly or indirectly powers the operation device.

40. An apparatus according to claim 38 or 39, wherein the control device controls the operation device to operate the restriction device.

41. An apparatus according to claim 40, wherein the operation device comprises hydraulic means and at least one valve for controlling a fluid flow in the hydraulic means.

42. An apparatus according to claim 41, wherein the control device comprises a wireless remote control for controlling the valve.

43. An apparatus according to claim 40, wherein the restriction device comprises hydraulic means and the operation device comprises a reservoir forming a fluid chamber with a variable volume connected to the hydraulic means, and the operation device is adapted to distribute fluid from the chamber to the hydraulic means by reduction of the volume of the chamber and to withdraw fluid from the hydraulic means to the chamber by expansion of the volume of the chamber.

44. An apparatus according to claim 38 or 40, wherein the operation device comprises a motor.

45. An apparatus according to claim 44, wherein the motor comprises a rotary motor, and the control device controls the rotary motor to rotate a desired number of revolutions.

46. An apparatus according to claim 44, wherein the motor comprises a linear motor.

47. An apparatus according to claim 44, wherein the motor comprises a hydraulic or pneumatic fluid motor, and the control device controls the fluid motor.

48. An apparatus according to claim 44, wherein the motor comprises an electric motor having electrically conductive parts made of plastics.

49. An apparatus according to any one of claims 1, 38-40, wherein the control device releases polarized energy from the source of energy.

50. An apparatus according to any one of claims 38-40, wherein the control device shifts polarity of the released energy to reverse the operation device.

51. An apparatus according to any one of claims 38-40, wherein the operation device comprises an electric motor and the released energy comprises electric energy.

52. An apparatus according to any one of claims 1, 6, 38-40 and 44, further comprising a reversing device implantable in the patient for reversing the restriction device.

53. An apparatus according to claim 52, wherein the control device controls the reversing device to reverse the restriction device.

54. An apparatus according to claim 52, wherein the reversing device comprises hydraulic means including a valve for shifting the flow direction of a fluid in the hydraulic means.

55. An apparatus according to claim 52, wherein the reversing device comprises a mechanical reversing device.

56. An apparatus according to claim 55, wherein the mechanical reversing device comprises a switch.

57. An apparatus according to claim 55, wherein the reversing device comprises a gearbox.

58. An apparatus according to claim 52, wherein the reversing device comprises a switch.

59. An apparatus according to claim 58, wherein the switch of the reversing device is operable by the released energy.

60. An apparatus according to claim 59, wherein the control device controls the operation of the switch of the reversing device by shifting polarity of the released energy supplied to the switch.

61. An apparatus according to claim 58, wherein the switch comprises an electric switch and the source of energy supplies electric energy for the operation of the switch.

62. An apparatus according to claim 58, wherein the operation device comprises a motor, and the reversing device reverses the motor.

63. An apparatus according to any of claims 1, 2, 6, 38-40, wherein the restriction device comprises hydraulic means and the operation device is adapted to conduct a hydraulic fluid in the hydraulic means.

64. An apparatus according to claim 63, wherein the operation device comprises a motor.

65. An apparatus according to claim 63 or 64, wherein the operation device comprises a fluid conduit connected to the hydraulic means of the restriction device, and a reservoir for fluid, the reservoir forming part of the conduit.

66. An apparatus according to claim 65, wherein the hydraulic means and conduit are devoid of any non-return valve.

67. An apparatus according to claim 66, wherein the reservoir forms a fluid chamber with a variable volume, and the operation device is adapted to distribute fluid from the chamber to the hydraulic means of the restriction device by reduction of the volume of the chamber and to withdraw fluid from the hydraulic means to the chamber by expansion of the volume of the chamber.

68. An apparatus according to any of the preceding claims, further comprising at least one implantable sensor for sensing at least one physical parameter of the patient.

69. An apparatus according to claim 68, wherein the sensor comprises a pressure sensor for directly or indirectly sensing as the physical parameter the pressure in the food passageway.

70. An apparatus according to claim 68 or 69, wherein the control device controls the restriction device in response to signals from the sensor.

71. An apparatus according to claim 70, wherein the control device comprises an internal control unit implantable in the patient, the internal control unit controlling the restriction device in response to signals from the sensor.

72. An apparatus according to claim 71, wherein the control device comprises an external control unit outside the patient's body, the external control unit controlling the restriction device in response to signals from the sensor.

73. An apparatus according to claim 72, wherein the external control unit stores information on the physical parameter sensed by the sensor and is manually operated to control the restriction device based on the stored information.

74. An apparatus according to any of claims 68-73, further comprising at least one implantable sender for sending information on the physical parameter sensed by the sensor.

75. An apparatus according to any of the preceding claims, further comprising an external data communicator intended to be outside the patient's body and an internal data communicator implantable in the patient for communicating with the external communicator, wherein the internal data communicator feeds data related to the patient back to the external data communicator or the external data communicator feeds data to the internal data communicator.

76. An apparatus according to claim 75, wherein the internal data communicator feeds data related to the restriction device.

77. An apparatus according to claim 75 or 76, wherein the implantable communicator feeds data related to at least one physical signal of the patient.

78. An apparatus according to any of the preceding claims, wherein the restriction device is adapted to control the cross-sectional area of the food passageway.

79. An apparatus according to claim 78, wherein the restriction device is operable to open and close the food passageway when implanted in the patient.

80. An apparatus according to claim 78 or 79, wherein the restriction device is adapted to steplessly control the cross-sectional area of the food passageway when implanted in the patient.

81. An apparatus according to any of the preceding claims, further comprising an implantable energy transforming device, wherein the control device is adapted to control the source of energy to release wireless electric energy and the energy transforming device is adapted to transform the electric energy into kinetic energy for operation of the restriction device.

82. An apparatus according to claim 81, wherein the restriction device is directly operated with the kinetic energy, as the energy transforming device transforms the electric energy into the kinetic energy.

83. An apparatus according to any of the preceding claims, wherein the restriction device is non-inflatable.

84. An apparatus according to any of the preceding claims, wherein the control device controls the source of energy to release energy for a determined time period.

85. An apparatus according to any of claims 1-83, wherein the control device controls the source of energy to release energy in a determined number of energy pulses.

86. An apparatus according to any of the preceding claims, wherein the control device is adapted to control the source of energy to release energy in a non-invasive manner.

87. An apparatus according to any of the preceding claims, wherein the control device comprises a wireless remote control for transmitting at least one wireless control signal for controlling the restriction device.

88. An apparatus according to claim 87, wherein the remote control is capable of obtaining information on the condition of the restriction device when the restriction device is implanted and to control the restriction device in response to the information.

89. An apparatus according to claim 87 or 88, wherein the wireless remote control comprises at least one external signal transmitter or transceiver and at least one internal signal receiver or transceiver implantable in the patient.

90. An apparatus according to claim 87 or 88, wherein the wireless remote control comprises at least one external signal receiver or transceiver and at least one internal signal transmitter or transceiver implantable in the patient.

91. An apparatus according to any of claims 87-90, wherein the remote control is capable of sending information related to the restriction device from inside the patient's body to the outside thereof.

92. An apparatus according to claim 91, wherein the remote control controls the restriction device in response to the information.

93. An apparatus according to any of claims 87-92, wherein the remote control transmits a carrier signal for carrying the control signal.

94. An apparatus according to claim 93, wherein the carrier signal is frequency, amplitude or frequency and amplitude modulated.

95. An apparatus according to claim 93 or 94, wherein the carrier signal is digital, analog or digital and analog.

96. An apparatus according to any of claims 93-95, wherein the control signal used with the carrier signal is frequency, amplitude or frequency and amplitude modulated.

97. An apparatus according to claim 87-96, wherein the control signal comprises an electric, magnetic or electric and magnetic field.

98. An apparatus according to any of claims 87-96, wherein the control signal is digital, analog or digital and analog.

99. An apparatus according to claim 98, wherein the remote control transmits an electromagnetic carrier wave signal for carrying the digital or analog control signal.

100. An apparatus according to any of claims 87-99, wherein the control signal is transmitted in pulses by the wireless remote control.

101. An apparatus according to any of claims 2, 15-17, 79-81, further comprising an implantable stabiliser for stabilising the energy released by the control device.

102. An apparatus according to claim 101, wherein the energy released by the control device comprises electric energy and the stabiliser comprises at least one capacitor.

103. An apparatus according to claim 15 or 16, wherein the control device is adapted to control the source of energy to release electric energy, and further comprising an implantable capacitor for producing the train of energy pulses from the released energy.

104. An apparatus according to claim 1, further comprising an adjustment device for adjusting the restriction device to change the size of the stoma opening, wherein the adjustment device is adapted to mechanically adjust the restriction device, or adapted to hydraulically adjust the restriction device by using hydraulic means which is devoid of hydraulic fluid of the kind having a viscosity that substantially increases when exposed to heat or a magnetic field.

105. An apparatus according to claim 38, wherein the operation device comprises an electrical operation device.

106. An apparatus according to claim 38, wherein the operation device is powered by magnetic energy, non-magnetic energy, electromagnetic energy, non-electromagnetic energy, kinetic energy, non-kinetic energy, thermal energy or non-thermal energy.

107. An apparatus according to claim 1, further comprising implantable electrical components including at least one voltage level guard.

108. An apparatus according to claim 1, further comprising implantable electrical components including a single voltage level guard.

109. An apparatus according to claim 107 or 108, wherein the electrical components are devoid of any current detector and/or charge level detector.

110. An apparatus according to any of claims 107-109, further comprising an implantable capacitor or accumulator, wherein the charge or discharge of the capacitor or accumulator is controlled by use of the voltage level guard.

111. An apparatus according to claim 15, wherein the released energy comprises electric energy and further comprising an implantable capacitor for producing the train of energy pulses.

112. An apparatus according to claim 110 or 111, wherein the capacitor has a capacity less than 0.1 pF.

113. An apparatus according to any of claims 1, 2, 15 and 16, further comprising an implantable motor or pump for operating the restriction device, wherein the control device is adapted to control the source of energy to directly power the motor or pump with the released energy.

114. An apparatus according to claim 15, wherein the wireless energy comprises electromagnetic waves excluding radio waves.

115. An apparatus according to any of claims 2, 15 and 16, further comprising an implantable motor or pump for operating the restriction device, wherein the control device is adapted to release wireless energy in the form of a magnetic field or electromagnetic waves for direct power of the motor or pump, as the wireless energy is being released.

116. An apparatus according to claim 115, wherein the pump is not a plunger type of pump.

117. An apparatus according to any of claims 2, 15 or 16, wherein the wireless energy comprises a signal.

118. An apparatus according to claim 1, further comprising an implantable energy transforming device for transforming wireless energy directly or indirectly into energy different than the wireless energy for operation of the restriction device.

119. An apparatus according to claim 118, wherein the energy transforming device transforms the wireless energy in the form of sound waves into electric energy for operation of the restriction device.

120. An apparatus according to claim 119, wherein the energy transforming device transforms the wireless energy in the form of sound waves directly into electric energy.

121. An apparatus according to claim 119 or 120, wherein the energy transforming device comprises a capacitor.

122. An apparatus according to claim 121, wherein the capacitor is adapted to produce electric pulses from the transformed electric energy.

123. An apparatus according to any of claims 118-122, further comprising an implantable motor or pump for oprating the restriction device, wherein the motor or pump is powered by the transformed energy.

124. An apparatus according to claim 1, wherein the restriction device is adjustable in a non-manual manner.

125. An apparatus according to any of the preceding claims, wherein the restriction device is embedded in a soft or gel-like material.

126. An apparatus according to any of the preceding claims, wherein the restriction device is embedded in a silicone material having hardness less than 20 Shore.

## Patentansprüche

1. Vorrichtung für die Behandlung von Sodbrennen und der Reflux-Krankheit, mit einem Restriktionselement, das in einen Patienten, der an Sodbrennen und an der Reflux-Krankheit leidet, implantierbar ist, zum Umgreifen des Magens nahe dem Mageneingang oder zum Umgreifen der Speiseröhre, um einen verengten Nahrungsdurchlass in den Magen oder in die Speiserohre zu bilden, wobei das Restriktionselement so betreibbar ist, dass es die Verengung des verengten Nahrungsdurchlasses ändert, wobei eine Quelle für Energie zur Verfügung gestellt ist und eine Steuervorrichtung, die von außerhalb des Körpers des Patienten betätigbar ist, zum Steuern der Quellen für Energie vorgesehen ist, um Energie zur Verwendung in Verbindung mit dem Betrieb des Restriktionselementes, wenn das Restriktionselement implantiert ist, abzugeben, wobei die Steuervorrichtung eine programmierbare interne Steuereinheit, die in den Patienten zum Steuern des Restriktionselementes implantierbar ist, und eine externe Steuereinheit, die außerhalb des Körpers des Patienten geplant ist, aufweist, wobei die interne Steuereinheit durch die externe Steuereinheit programmierbar ist und wobei die externe Steuereinheit dazu ausgelegt ist, in die interne Steuereinheit Daten zu laden oder die interne Steuereinheit zu steuern, gemäß einem Arztmodus, für den nur ein Arzt die Rechte hat, **dadurch gekennzeichnet, dass** die externe Steuereinheit weiterhin entsprechend einem Patientenmodus verwendet werden kann, der für den Patienten erlaubt ist, wobei bei der Verwendung der Patientenmodus es dem Patienten erlaubt, auf erste Funktionen der internen Steuereinheit zu greifen und der Arztmodus es dem Arzt erlaubt, auf zweite Funktionen der internen Steuereinheit zuzugreifen.

2. Vorrichtung nach Anspruch 1, bei der die Quelle für Energie außerhalb des Körpers des Patienten geplant ist, wenn das Restriktionselement implantiert ist, und die Steuervorrichtung dazu ausgelegt ist, die externe Quelle für Energie so zu steuern, dass sie drahtlose Energie zur Verwendung in Verbindung mit dem Betrieb des Restriktionselementes abgibt.

3. Vorrichtung nach Anspruch 1, bei der die interne Steuereinheit zum Steuern des Restriktionselementes über die Zeit programmierbar ist.

4. Vorrichtung nach Anspruch 3, bei der die interne Steuereinheit das Restriktionselement über die Zeit entsprechend einem Aktivitätenplan-Programm steuert.

5. Vorrichtung nach Anspruch 3, bei der die interne Steuereinheit einen Mikroprozessor aufweist.

6. Vorrichtung nach Anspruch 1, bei der die Quelle für Energie in den Patienten implantierbar ist.

7. Vorrichtung nach Anspruch 6, bei der die implantierbare Quelle für Energie wenigstens einen Akkumulator, wenigstens einen Kondensator oder wenigstens eine wieder aufladbare Batterie oder eine Kombination aus wenigstens einem Kondensator und wenigstens einer wieder aufladbaren Batterie aufweist.

8. Vorrichtung nach Anspruch 7, bei der die implantierbare Quelle für Energie eine Quelle für elektrische Energie aufweist.

9. Vorrichtung nach Anspruch 8, bei der die Quelle für elektrische Energie einen Akkumulator oder eine Batterie mit einer Lebensdauer von wenigstens 10 Jahren aufweist.

10. Vorrichtung nach Anspruch 2, weiter mit einer Energiespeichervorrichtung, die in den Patienten implantierbar ist, um die drahtlose Energie zu speichern, die von der externen Quelle für Energie abgegeben worden ist.

11. Vorrichtung nach Anspruch 10, bei der die Energiespeichervorrichtung einen Akkumulator aufweist.

12. Vorrichtung nach Anspruch 11, bei der der Akkumulator einen elektrischen Akkumulator aufweist.

13. Vorrichtung nach Anspruch 12, bei der elektrische Akkumulator wenigstens einen Kondensator oder wenigstens eine wieder aufladbare Batterie oder eine Kombination aus wenigstens einem Kondensator und wenigstens einer wieder aufladbaren Batterie aufweist.

14. Vorrichtung nach Anspruch 1 oder 2, weiter mit einer Batterie, die in den Patienten implantierbar ist, zum Liefern von elektrischer Energie an implantierbare, elektrische Energie verbrauchende Komponenten der Vorrichtung.

15. Vorrichtung nach Anspruch 2, bei der die Steuervorrichtung dazu ausgelegt ist, die externe Quelle für Energie zu steuern, so dass sie drahtlose Energie für direkte Verwendung in Verbindung mit dem Betrieb des Restriktionselementes abgibt.

16. Vorrichtung nach Anspruch 15, bei der die Steuervorrichtung dazu ausgelegt ist, die externe Quelle für Energie so zu steuern, dass sie diskontinuierlich drahtlose Energie in der Form eines Zuges aus Energiepulsen für direkte Verwendung in Verbindung mit dem Betrieb des Restriktionselementes abgibt.

17. Vorrichtung nach Anspruch 15 oder 16, bei der Restriktionselement durch Einsatz der abgegebenen drahtlosen Energie nicht magnetisch, nicht thermisch oder nicht mechanisch betreibbar ist.

18. Vorrichtung nach Anspruch 1 oder Anspruch 2, weiter mit einem Schalter, der in den Patienten implantierbar ist, zum direkten oder indirekten Umschalten des Betriebes des Restriktionselementes.

19. Vorrichtung nach Anspruch 18, weiter mit einer internen Quelle für Energie, die in den Patienten implantierbar ist, zum Liefern von Energie für den Betrieb des Restriktionselementes, wobei der Schalter direkt oder indirekt die Lieferung von Energie von der internen Quelle für Energie beeinflusst.

20. Vorrichtung nach Anspruch 19, bei der der Schalter zwischen einem "aus"-Modus, in dem die interne Quelle für Energie nicht im Einsatz ist, und einem "ein"-Modus, in dem die interne Quelle für Energie Energie für den Betrieb des Restriktionselementes liefert, schaltet.

21. Vorrichtung nach Anspruch 2 und 20, bei der der Schalter durch die drahtlose Energie betätigbar ist, die von der externen Quelle für Energie abgegeben wird.

22. Vorrichtung nach Anspruch 21, bei der die Steuervorrichtung die externe Quelle für Energie steuert, um die drahtlose Energie abzugeben.

23. Vorrichtung nach einem der Ansprüche 1, 2 und 22, bei der die Steuervorrichtung eine drahtlose Fernsteuerung aufweist.

24. Vorrichtung nach Anspruch 19, bei der die Steuervorrichtung eine drahtlose Fernsteuerung zum Steuern der internen Quelle für Energie aufweist.

25. Vorrichtung nach Anspruch 24, bei der der Schalter durch die drahtlose Energie von der externen Quelle für Energie betreibbar ist, um zwischen einem "aus"-Modus, in dem die interne Quelle für Energie und die Fernsteuerung nicht im Einsatz sind, und einem "Bereitschaft"-Modus, in dem die Fernsteuerung die interne Quelle für Energie so steuern kann, dass sie Energie für den Betrieb des Restriktionselementes liefert, zu schalten.

26. Vorrichtung nach Anspruch 19, weiter mit einer Energieumwandelvorrichtung, die in den Patienten implantierbar ist, zum Umwandeln der drahtlosen Energie in speicherbare Energie, und einer Energiespeichervorrichtung, die in den Patienten implantierbar ist, zum Speichern der speicherbaren Energie.

27. Vorrichtung nach Anspruch 26, bei der der Schalter durch Energie von der implantierbaren Energiespeichervorrichtung betreibbar ist, um zwischen einem "aus"-Modus, in dem die interne Quelle für Energie nicht im Einsatz ist, und einem "ein"-Modus, in dem die interne Quelle für Energie Energie für den Betrieb des Restriktionselementes liefert, zu schalten.

28. Vorrichtung nach Anspruch 27, bei der die Steuervorrichtung die Energiespeichervorrichtung so steuert, dass sie den Schalter betätigt.

29. Vorrichtung nach Anspruch 28, bei der die Steuervorrichtung eine drahtlose Fernsteuerung aufweist.

30. Vorrichtung nach Anspruch 29, weiter mit einer Energieumwandelvorrichtung, die in den Patienten implantierbar ist, zum Umwandeln der drahtlosen Energie in speicherbare Energie, wobei die interne Quelle für Energie in der Lage ist, die speicherbare Energie zu speichern.

31. Vorrichtung nach Anspruch 30, bei der der Schalter aus einem "aus"-Modus, in dem die interne Quelle für Energie nicht im Einsatz ist, in einen "ein"-Modus, in dem die Quelle für Energie Energie für den Betrieb des Restriktionselementes liefert, schaltet.

32. Vorrichtung nach Anspruch 31, bei der die Steuervorrichtung den Schalter steuert, so dass er zwischen dem "ein"- und dem "aus"-Modus schaltet.

33. Vorrichtung nach Anspruch 32, bei der die Steuervorrichtung eine drahtlose Fernsteuerung aufweist.

34. Vorrichtung nach Anspruch 19, bei der die interne Quelle für Energie eine Quelle für elektrische Energie aufweist.

35. Vorrichtung nach Anspruch 34, bei der die Quelle für elektrische Energie wenigstens einen Akkumulator, wenigstens einen Kondensator oder wenigstens eine wieder aufladbare Batterie oder eine Kombination aus wenigstens einem Kondensator und wenigstens einer wieder aufladbaren Batterie aufweist.

36. Vorrichtung nach Anspruch 34, bei der die Quelle für elektrische Energie einen Akkumulator oder eine Batterie mit einer Lebensdauer von wenigstens 10 Jahren aufweist.

37. Vorrichtung nach Anspruch 1, bei der die Quelle für Energie eine Quelle für chemische Energie aufweist.

38. Vorrichtung nach einem der Ansprüche 1, 2, 6 oder 18, weiter mit einer Betreibervorrichtung, die in den Patienten zum Betreiben des Restriktionselementes implantierbar ist.

39. Vorrichtung nach Anspruch 2 oder 15, weiter mit einer Betreibervorrichtung, die in den Patienten, zum Betreiben des Restriktionselementes implantierbar ist, wobei die drahtlose Energie die Betreibervorrichtung direkt oder indirekt mit Energie versorgt.

40. Vorrichtung nach Anspruch 38 oder 39, bei der die Steuervorrichtung die Betreibervorrichtung so steuert, daß sie das Restriktionselement betreibt.

41. Vorrichtung nach Anspruch 40, bei der die Betreibervorrichtung eine hydraulische Einrichtung und wenigstens ein Ventil zum Steuern eines Fluidstromes in der hydraulischen Einrichtung aufweist.

42. Vorrichtung nach Anspruch 41, bei der die Steuervorrichtung eine drahtlose Fernsteuerung zum Steuern des Ventils aufweist.

43. Vorrichtung nach Anspruch 40, bei der das Restriktionselement eine hydraulische Einrichtung aufweist und die Betreibervorrichtung einen Behälter aufweist, der eine Fluidkammer mit einem variablen Volumen bildet, die mit der hydraulischen Einrichtung verbunden ist, und die Betreibervorrichtung dazu ausgelegt ist, Fluid aus der Kammer durch Verringerung des Volumens der Kammer in die hydraulische Einrichtung zu verteilen und Fluid aus der hydraulischen Einrichtung durch Vergrößerung des Volumens der Kammer in die Kammer zu ziehen.

44. Vorrichtung nach Anspruch 38 oder 40, bei der die Betreibervorrichtung einen Motor aufweist.

45. Vorrichtung nach Anspruch 44, bei der der Motor einen Drehmotor aufweist und die Steuervorrichtung den Motor so steuert, daß er sich eine gewünschte Anzahl von Umdrehungen dreht.

46. Vorrichtung nach Anspruch 44, bei der der Motor einen Linearmotor aufweist.

47. Vorrichtung nach Anspruch 44, bei der der Motor einen hydraulischen oder pneumatischen Fluidmotor aufweist und die Steuervorrichtung den Fluidmotor steuert.

48. Vorrichtung nach Anspruch 44, bei der der Motor einen elektrischen Motor mit elektrisch leitenden Teilen, die aus Kunststoff hergestellt sind, aufweist.

49. Vorrichtung nach einem der Ansprüche 1, 38 - 40, bei der die Steuervorrichtung polarisierte Energie aus der Quelle für Energie abgibt.

50. Vorrichtung nach einem der Ansprüche 38 - 40, bei der die Steuervorrichtung die Polarität der abgegebenen Energie ändert, um die Betreibervorrichtung umgekehrt zu betreiben.

51. Vorrichtung nach einem der Ansprüche 38 - 40, bei der die Betreibervorrichtung einen elektrischen Motor aufweist und die abgegebene Energie elektrische Energie aufweist.

52. Vorrichtung nach einem der Ansprüche 1, 6, 38 - 40 und 44, weiter mit einer invertierenden Vorrichtung, die in den Patienten implantierbar ist, um den Betrieb des Restriktionselementes umzukehren.

53. Vorrichtung nach Anspruch 52, bei der die Steuervorrichtung die invertierende Vorrichtung so steuert, dass sie das Restriktionselement umgekehrt betreibt.

54. Vorrichtung nach Anspruch 52, bei der die invertierende Vorrichtung eine hydraulische Einrichtung, die ein Ventil umfasst zum Ändern der Strömungsrichtung eines Fluides in der hydraulischen Einrichtung aufweist.

55. Vorrichtung nach Anspruch 52, bei der die invertierende Vorrichtung eine mechanische invertierende Vorrichtung aufweist.

56. Vorrichtung nach Anspruch 55, bei der die mechanische invertierende Vorrichtung einen Schalter aufweist.

57. Vorrichtung nach Anspruch 55, bei der die invertierende Vorrichtung einen Getriebekörper aufweist.

58. Vorrichtung nach Anspruch 52, bei der die invertierende Vorrichtung einen Schalter aufweist.

59. Vorrichtung nach Anspruch 58, bei der der Schalter der invertierenden Vorrichtung durch die abgegebene Energie betreibbar ist.

60. Vorrichtung nach Anspruch 59, bei der die Steuervorrichtung den Betrieb des Schalters der invertierenden Vorrichtung steuert, in dem die Polarität der abgegebenen Energie, die an den Schalter geliefert wird, geändert wird.

61. Vorrichtung nach Anspruch 58, bei der der Schalter einen elektrischen Schalter aufweist und die Quelle für Energie elektrische Energie für den Betrieb des Schalters liefert.

62. Vorrichtung nach Anspruch 58, bei der die Betreibervorrichtung einen Motor aufweist und die invertierende Vorrichtung den Motor umgekehrt betreibt.

63. Vorrichtung nach einem der Ansprüche 1, 2, 6, 38 - 40, bei der das Restriktionselement eine hydraulische Einrichtung aufweist und die Betreibervorrichtung dazu ausgelegt ist, ein hydraulisches Fluid in die hydraulische Einrichtung zu leiten.

64. Vorrichtung nach Anspruch 63, bei der die Betreibervorrichtung einen Motor aufweist.

65. Vorrichtung nach Anspruch 63 oder 64, bei der die Betreibervorrichtung eine Fluidleitung, die mit der hydraulischen Einrichtung des Restriktionselementes verbunden ist, und einen Behälter für Fluid aufweist, wobei der Behälter einen Teil der Leitung bildet.

66. Vorrichtung nach Anspruch 65, bei der die hydraulische Einrichtung und die Leitung kein Einwegventil enthalten.

67. Vorrichtung nach Anspruch 66, bei der der Behälter eine Fluidkammer mit einem variablen Volumen bildet und die Betreibervorrichtung dazu ausgelegt ist, Fluid aus der Kammer durch Verringerung des Volumens der Kammer in die hydraulische Einrichtung des Restriktionselementes zu verteilen und Fluid aus der hydraulischen Einrichtung durch Vergrößerung des Volumens der Kammer in die Kammer zu ziehen.

68. Vorrichtung nach einem der vorangehenden Ansprüche, weiter mit wenigstens einem implantierbaren Sensor zum Abfühlen wenigstens eines physikalischen Parameters des Patienten.

69. Vorrichtung nach Anspruch 68, bei der der Sensor einen Drucksensor zum direkten oder indirekten Abfühlen des Druckes in dem Nahrungsdurchlass als den physikalischen Parameter aufweist.

70. Vorrichtung nach Anspruch 68 oder 69, bei der die Steuervorrichtung das Restriktionselement als Antwort auf Signale von dem Sensor steuert.

71. Vorrichtung nach Anspruch 70, bei der die Steuervorrichtung eine interne Steuereinheit aufweist, die in den Patienten implantierbar ist, wobei die interne Steuereinheit das Restriktionselement als Antwort auf Signale von dem Sensor steuert.

72. Vorrichtung nach Anspruch 71, bei der die Steuervorrichtung eine externe Steuereinheit außerhalb des Körpers des Patienten aufweist, wobei die externe Steuereinheit das Restriktionselement als Antwort auf Signale von dem Sensor steuert.

73. Vorrichtung nach Anspruch 72, bei der die externe Steuereinheit Information über den physikalischen Parameter, der von dem Sensor abgefühlt wird, speichert und manuell betrieben wird, um das Restriktionselement basierend auf der gespeicherten Information zu steuern.

74. Vorrichtung nach einem der Ansprüche 68 - 73, weiter mit wenigstens einem implantierbaren Senders zum Senden von Informationen über den physikalischen Parameter, der von dem Sensor abgefühlt wird.

75. Vorrichtung nach einem der vorangehenden Ansprüche, weiter mit einem externen Datenkommunikator, der außerhalb des Körpers des Patienten geplant ist, und einem internen Datenkommunikator, der in den Patienten implantierbar ist, zum Kommunizieren mit dem externen Kommunikator, wobei der interne Datenkommunikator Daten, die den Patienten betreffen, zurück an den externen Datenkommunikator gibt oder der externe Datenkommunikator Daten an den internen Datenkommunikator gibt.

76. Vorrichtung nach Anspruch 75, bei der der interne Datenkommunikator Daten, die das Restriktionselement betreffen, weitergibt.

77. Vorrichtung nach Anspruch 75 oder 76, bei der der implantierbare Kommunikator Daten weitergibt, die sich auf wenigstens ein physikalisches Signal des Patienten beziehen.

78. Vorrichtung nach einem der vorangehenden Ansprüche, bei der das Restriktionselement dazu ausgelegt ist, die Querschnittsfläche des Nahrungsdurchlasses zu steuern.

79. Vorrichtung nach Anspruch 78, in der das Restriktionselement so betreibbar ist, dass es den Nahrungsdurchlass öffnet oder schließt, wenn es in dem Patienten implantiert ist.

80. Vorrichtung nach Anspruch 78 oder 79, bei der das Restriktionselement dazu ausgelegt ist, die Querschnittsfläche des Nahrungsdurchlasses stufenlos zu steuern, wenn es in dem Patienten implantiert ist.

81. Vorrichtung nach einem der vorangehenden Ansprüche, weiter mit einer implantierbaren Energieumwandelvorrichtung, wobei die Steuervorrichtung dazu ausgelegt ist, die Quelle für Energie so zu steuern, dass drahtlose elektrische Energie abgegeben wird, und die Energieumwandelvorrichtung dazu ausgelegt ist, die elektrische Energie in kinetische Energie für den Betrieb des Restriktionselementes umzuwandeln.

82. Vorrichtung nach Anspruch 81, bei der das Restriktionselement direkt mit der kinetischen Energie betrieben wird, wenn die Energieumwandelvorrichtung die elektrische Energie in die kinetische Energie umwandelt.

83. Vorrichtung nach einem der vorangehenden Ansprüche, bei der das Restriktionselement nicht aufweitbar ist.

84. Vorrichtung nach einem der vorangehenden Ansprüche, bei der die Steuervorrichtung die Quelle für Energie so steuert, dass sie Energie über eine vorbestimmte Zeitdauer abgibt.

85. Vorrichtung nach einem der Ansprüche 1 - 83, bei der die Steuervorrichtung die Quelle für Energie so steuert, dass sie Energie in einer vorbestimmten Anzahl von Energiepulsen abgibt.

86. Vorrichtung nach einem der vorangehenden Ansprüche, bei der die Steuervorrichtung dazu ausgelegt ist, die Quelle für Energie so zu steuern, dass sie Energie in einer nicht invasiven Weise abgibt.

87. Vorrichtung nach einem der vorangehenden Ansprüche, bei der die Steuervorrichtung eine drahtlose Fernsteuerung zum Senden wenigstens eines drahtlosen Steuersignals zum Steuern des Restriktionselementes aufweist.

88. Vorrichtung nach Anspruch 87, bei der die Fernsteuerung in der Lage ist, Information über den Zustand des Restriktionselementes zu erhalten, wenn das Restriktionselement implantiert ist, und das Restriktionselement als Antwort auf die Information zu steuern.

89. Vorrichtung nach Anspruch 87 oder 88, bei der die drahtlose Fernsteuerung wenigstens einen externen Signalsender oder Transceiver und wenigstens einen internen Signalempfänger oder Transceiver, der in den Patienten implantierbar ist, aufweist.

90. Vorrichtung nach Anspruch 87 oder 88, bei der die drahtlose Fernsteuerung wenigstens einen externen Signalempfänger oder Transceiver und wenigstens einen internen Signalsender oder Transceiver, der in den Patienten implantierbar ist, aufweist.

91. Vorrichtung nach einem der Ansprüche 87 - 90, bei der die Fernsteuerung in der Lage ist, Information, die das Restriktionselement betrifft, von innerhalb des Körpers des Patienten nach außerhalb zu senden.

92. Vorrichtung nach Anspruch 91, bei der die Fernsteuerung das Restriktionselement als Antwort auf die Information steuert.

93. Vorrichtung nach einem der Ansprüche 87 - 92, bei der die Fernsteuerung ein Trägersignal zum Transportieren des Steuersignals sendet.

94. Vorrichtung nach Anspruch 93, bei der das Trägersignal frequenz-, amplituden- oder frequenz- und amplitudenmoduliert ist.

95. Vorrichtung nach Anspruch 93 oder 94, bei der das Trägersignal digital, analog oder digital und analog ist.

96. Vorrichtung nach einem der Ansprüche 93 - 95, bei der das Steuersignal, das mit dem Trägersignal verwendet wird, frequenz-, amplituden- oder frequenz- und amplitudenmoduliert ist.

97. Vorrichtung nach Anspruch 87 - 96, bei der das Steuersignal ein elektrisches, ein magnetisches oder ein elektrisches und magnetisches Feld aufweist.

98. Vorrichtung nach einem der Ansprüche 87 - 96, bei der das Steuersignal digital, analog oder digital und analog ist.

99. Vorrichtung nach Anspruch 98, bei der die Fernsteuerung ein elektromagnetisches Trägerwellensignal zum Transportieren des digitalen oder analogen Steuersignals sendet.

100. Vorrichtung nach einem der Ansprüche 87 - 99, bei der das Steuersignal von der drahtlosen Fernsteuerung in Pulsen gesendet wird.

101. Vorrichtung nach einem der Ansprüche 2, 15 - 17, 79 - 81, weiter mit einem implantierbaren Stabilisator zum Stabilisieren der Energie, die von der Steuervorrichtung abgegeben wird.

102. Vorrichtung nach Anspruch 101, bei der die Energie, die von der Steuervorrichtung abgegeben wird, elektrische Energie aufweist und der Stabilisator wenigstens einen Kondensator aufweist.

103. Vorrichtung nach Anspruch 15 oder 16, bei der die Steuervorrichtung dazu ausgelegt ist, die Quelle für Energie so zu steuern, dass sie Energie abgibt, und weiter mit einem implantierbaren Kondensator zum Erzeugen des Zuges aus Energiepulsen aus der abgegebenen Energie.

104. Vorrichtung nach Anspruch 1, weiter mit einer eine Stellvorrichtung zum Einstellen des Restriktionselementes, um die Größe der Magenöffnung zu ändern, wobei die Einstellvorrichtung dazu ausgelegt ist, das Restriktionselement mechanisch einzustellen, oder dazu ausgelegt ist, das Restriktionselement hydraulisch einzustellen, indem eine hydraulische Einrichtung verwendet wird, die frei von hydraulischem Fluid der Art ist, das eine Viskosität hat, welche wesentlich anwächst, wenn sie Wärme oder einem Magnetfeld ausgesetzt ist.

105. Vorrichtung nach Anspruch 38, bei der die Betreibervorrichtung eine elektrische Betreibervorrichtung aufweist.

106. Vorrichtung nach Anspruch 38, bei der die Betreibervorrichtung mit magnetischer Energie, nichtmagnetischer Energie, elektromagnetischer Energie, nichtelektromagnetischer Energie, kinetischer Energie, nichtkinetischer Energie, thermischer Energie oder nichtthermischer Energie versorgt wird.

107. Vorrichtung nach Anspruch 1, weiter mit implantierbaren elektrischen Komponenten, die wenigstens einen Spannungsschutz umfassen.

108. Vorrichtung nach Anspruch 1, weiter mit implantierbaren elektrischen Komponenten, die einen einzigen Spannungsschutz umfassen.

109. Vorrichtung nach Anspruch 107 oder 108, bei der die elektrischen Komponenten frei von irgendeinem Stromdetektor und/oder Ladungspegeldetektor sind.

110. Vorrichtung nach einem der Ansprüche 107 - 109, weiter mit einem implantierbaren Kondensator oder Akkumulator, wobei das Laden Entladen des Kondensators oder Akkumulators durch Einsatz des Spannungsschutzes gesteuert wird.

111. Vorrichtung nach Anspruch 15, bei der die abgegebene Energie elektrische Energie aufweist und weiter mit einem implantierbaren Kondensator zum Erzeugen des Zuges aus Energiepulsen.

112. Vorrichtung nach Anspruch 110 oder 111, bei der der Kondensator eine Kapazität geringer als 0.1 pF hat.

113. Vorrichtung nach einem der Ansprüche 1, 2, 15 und 16, weiter mit einem implantierbaren Motor oder einer Pumpe zum Betreiben des Restriktionselementes, wobei die Steuervorrichtung dazu ausgelegt ist, die Quelle für Energie zu steuern, um den Motor oder die Pumpe mit der abgegebenen Energie direkt zu versorgen.

114. Vorrichtung nach Anspruch 15, bei der die drahtlose Energie elektromagnetische Wellen aufweist, wobei Funkwellen ausgeschlossen sind.

115. Vorrichtung nach einem der Ansprüche 2, 15 und 16, weiter mit einem implantierbaren Motor oder einer Pumpe zum Betreiben des Restriktionselementes, wobei die Steuervorrichtung dazu ausgelegt ist, drahtlose Energie in der Form eines Magnetfeldes oder elektromagnetischer Wellen zum direkten Versorgen des Motors oder der Pumpe abzugeben, wenn die drahtlose Energie abgegeben wird.

116. Vorrichtung nach Anspruch 115, bei der die Pumpe keine Pumpe vom Tauchkolbentyp ist.

117. Vorrichtung nach einem der Ansprüche 2, 15 oder 16, bei der die drahtlose Energie ein Signal aufweist.

118. Vorrichtung nach Anspruch 1, weiter mit einer implantierbaren Energieumwandelvorrichtung zum Umwandeln von drahtloser Energie direkt oder indirekt in Energie, die sich von der drahtlosen unterscheidet, für den Betrieb des Restriktionselementes.

119. Vorrichtung nach Anspruch 118, bei der die Energieumwandelvorrichtung die drahtlose Energie in der Form von Schallwellen in elektrische Energie zum Betrieb des Restriktionselementes umwandelt.

120. Vorrichtung nach Anspruch 119, bei der die Energieumwandelvorrichtung die drahtlose Energie in der Form von Schallwellen direkt in elektrische Energie umwandelt.

121. Vorrichtung nach Anspruch 119 oder 120, bei der die Energieumwandelvorrichtung einen Kondensator aufweist.

122. Vorrichtung nach Anspruch 121, bei der der Kondensator dazu ausgelegt ist, elektrische Pulse aus der umgewandelten elektrischen Energie zu erzeugen.

123. Vorrichtung nach einem der Ansprüche 118 - 122, weiter mit einem implantierbaren Motor oder einer Pumpe zum Betreiben des Restriktionselementes, wobei der Motor oder die Pumpe mit der umgewandelten Energie versorgt wird.

124. Vorrichtung nach Anspruch 1, bei der das Restriktionselement nicht von Hand einstellbar ist.

125. Vorrichtung nach einem der vorangehenden Ansprüche, bei der das Restriktionselement in ein weiches oder gelartiges Material eingebettet ist.

126. Vorrichtung nach einem der vorangehenden Ansprüche, bei der das Restriktionselement in ein Silikonmaterial mit einer Härte von weniger als 20 Shore eingebettet ist.

## Revendications

1. Appareil de traitement des reflux et brûlures d'estomac, comprenant un dispositif de restriction implantable dans un patient, qui souffre de la maladie des reflux gastro-oesophagiens et de brûlures d'estomac, pour mettre en prise l'estomac près de l'orifice oesophagien ou mettre en prise l'oesophage, afin de former un passage restreint pour les aliments dans l'estomac ou l'oesophage, le dispositif de restriction pouvant être actionné, de façon à modifier la restriction du passage restreint pour aliments, dans lequel une source d'énergie est ménagée, et un dispositif de commande pouvant être actionné de l'extérieur du corps du patient est fourni pour commander la source d'énergie permettant de libérer de l'énergie, à utiliser dans le cadre de l'utilisation du dispositif de restriction, quand ledit dispositif de restriction est implanté, dans lequel le dispositif de commande comprend une unité de commande interne programmable implantable dans le patient, pour commander le dispositif de restriction et une unité de commande externe destinée à rester hors du corps du patient. L'unité de commande interne est programmable par l'unité de commande externe et l'unité de commande externe est adaptée pour charger l'unité de commande interne avec des données, ou pour commander l'unité de commande interne, conformément au mode docteur uniquement autorisé pour un médecin, l'unité de commande externe pouvant être en outre utilisée conformément à un mode patient autorisé pour le patient, dans lequel, en cours d'utilisation, le mode patient permet audit patient d'accéder aux premières fonctions de l'unité de commande interne et le mode docteur permet audit médecin d'accéder aux secondes fonctions de l'unité de commande interne.

2. Appareil selon la revendication 1, dans lequel la source d'énergie est destinée à être extérieure au corps du patient quand le dispositif de restriction y est implanté, et le dispositif de commande est adapté pour commander la source externe d'énergie afin de libérer une énergie sans fil, à utiliser dans le cadre du fonctionnement du dispositif de restriction.

3. Appareil selon la revendication 1, dans lequel l'unité de commande interne est programmable pour contrôler le dispositif de restriction au fil du temps.

4. Appareil selon la revendication 3, dans lequel l'unité de commande interne commande le dispositif de restriction au fil du temps, conformément à un programme d'activité.

5. Appareil selon la revendication 3, dans lequel l'unité de commande interne comprend un microprocesseur.

6. Appareil selon la revendication 1, dans lequel la source d'énergie est implantable dans le patient.

7. Appareil selon la revendication 6, dans lequel la source implantable d'énergie comprend au moins un accumulateur, au moins un condensateur ou au moins une batterie rechargeable, ou une combinaison d'au moins un condensateur et d'au moins une batterie rechargeable.

8. Appareil selon la revendication 7, dans lequel la source implantable d'énergie comprend une source d'énergie électrique.

9. Appareil selon la revendication 8, dans lequel la source d'énergie électrique comprend un accumulateur, ou une batterie ayant une durée de vie d'au moins 10 jours.

10. Appareil selon la revendication 2, comprenant en outre un dispositif de stockage d'énergie implantable dans le patient, afin de stocker l'énergie sans fil libérée de la source externe d'énergie.

11. Appareil selon la revendication 10, dans lequel le dispositif de stockage d'énergie comprend un accumulateur.

12. Appareil selon la revendication 11, dans lequel l'accumulateur comprend un accumulateur électrique.

13. Appareil selon la revendication 12, dans lequel l'accumulateur électrique comprend au moins un condensateur ou au moins une batterie rechargeable, ou une combinaison d'au moins un condensateur et d'au moins une batterie rechargeable.

14. Appareil selon la revendication 1 ou 2, comprenant en outre une batterie implantable dans le patient pour fournir de l'énergie électrique aux composants consommant de l'énergie électrique implantables de l'appareil.

15. Appareil selon la revendication 2, dans lequel le dispositif de commande est adapté pour commander la source d'énergie externe, afin de libérer l'énergie sans fil, pour une utilisation directe en connexion avec le fonctionnement du dispositif de restriction.

16. Appareil selon la revendication 15, dans lequel le dispositif de commande est adapté pour commander la source d'énergie externe, afin de libérer par intermittence l'énergie sans fil, sous la forme d'un train d'impulsions énergétiques, à utiliser directement dans le cadre du fonctionnement du dispositif de restriction.

17. Appareil selon la revendication 15 ou 16, dans lequel le dispositif de restriction peut être actionné de manière non magnétique, non thermique ou non mécanique en utilisant ladite énergie sans fil libérée.

18. Appareil selon les revendications 1 ou 2, comprenant en outre un commutateur implantable dans le patient, afin de commuter directement ou indirectement le fonctionnement du dispositif de restriction.

19. Appareil selon la revendication 18, comprenant en outre une source d'énergie interne implantable dans le patient pour fournir de l'énergie pour l'utilisation du dispositif de restriction, dans lequel le commutateur affecte directement ou indirectement l'alimentation d'énergie depuis la source d'énergie interne.

20. Appareil selon la revendication 19, dans lequel le commutateur permet la commutation entre un mode "off", où la source d'énergie interne n'est pas utilisée, et un mode "on", où la source d'énergie interne fournit de l'énergie pour le fonctionnement du dispositif de restriction.

21. Appareil selon la revendication 2 et 20, dans lequel le commutateur peut être actionné par l'énergie sans fil libérée depuis la source d'énergie externe.

22. Appareil selon la revendication 21, dans lequel le dispositif de commande commande la source d'énergie externe, afin de libérer l'énergie sans fil.

23. Appareil selon l'une quelconque des revendications 1, 2 et 22, dans lequel le dispositif de commande comprend une commande à distance sans fil.

24. Appareil selon la revendication 19, dans lequel le dispositif de commande comprend une commande à distance sans fil pour commander la source d'énergie interne.

25. Appareil selon la revendication 24, dans lequel le commutateur peut être actionné par l'énergie sans fil provenant de la source externe d'énergie, pour permettre une commutation entre un mode "off", dans lequel la source d'énergie interne et la commande à distance ne sont pas utilisées, et un mode "standby", dans lequel la commande à distance est autorisée à commander la source interne d'énergie, afin de fournir de l'énergie pour le fonctionnement du dispositif de restriction.

26. Appareil selon la revendication 19, comprenant en outre un dispositif de transformation d'énergie implantable dans le patient, destiné à transformer l'énergie sans fil en énergie stockable et un dispositif de stockage d'énergie implantable dans le patient pour stocker l'énergie stockable.

27. Appareil selon la revendication 26, dans lequel le commutateur peut fonctionner par le biais de l'énergie provenant du dispositif de stockage d'énergie implantable, afin de permettre une commutation entre un mode "off", dans lequel la source d'énergie interne n'est pas utilisée, et un mode "on", dans lequel la source d'énergie interne fournit de l'énergie pour le fonctionnement du dispositif de restriction.

28. Appareil selon la revendication 27, dans lequel le dispositif de commande commande le dispositif de stockage d'énergie pour faire fonctionner le commutateur.

29. Appareil selon la revendication 28, dans lequel le dispositif de commande comprend une commande à distance sans fil.

30. Appareil selon la revendication 29, comprenant en outre un dispositif de transformation d'énergie implantable dans le patient, afin de transformer l'énergie sans fil en énergie stockable, dans lequel la source d'énergie interne est capable de stocker l'énergie stockable.

31. Appareil selon la revendication 30, dans lequel le commutateur passe d'un mode "off", dans lequel la source d'énergie interne n'est pas utilisée, à un mode "on", dans lequel la source d'énergie interne fournit de l'énergie pour le fonctionnement du dispositif de restriction.

32. Appareil selon la revendication 31, dans lequel le dispositif de commande commande le commutateur, afin qu'il permette la commutation entre les modes "on" et "off".

33. Appareil selon la revendication 32, dans lequel le dispositif de commande comprend une commande à distance sans fil.

34. Appareil selon la revendication 19, dans lequel la source d'énergie interne comprend une source d'énergie électrique.

35. Appareil selon la revendication 34, dans lequel la source d'énergie électrique comprend au moins un accumulateur, au moins un condensateur ou au moins une batterie rechargeable, ou une combinaison d'au moins un condensateur et d'au moins une batterie rechargeable.

36. Appareil selon la revendication 34, dans lequel la source d'énergie électrique comprend un accumulateur ou une batterie ayant une durée de vie d'au moins 10 ans.

37. Appareil selon la revendication 1, dans lequel la source d'énergie comprend une source d'énergie chimique.

38. Appareil selon l'une quelconque des revendications 1, 2, 6 ou 18, comprenant en outre un dispositif de fonctionnement implantable dans le patient pour faire fonctionner le dispositif de restriction.

39. Appareil selon la revendication 2 ou 15, comprenant en outre un dispositif de fonctionnement implantable dans le patient pour faire fonctionner le dispositif de restriction, dans lequel l'énergie sans fil alimente directement ou indirectement le dispositif de fonctionnement.

40. Appareil selon les revendications 38 ou 39, dans lequel le dispositif de commande commande le dispositif de fonctionnement pour qu'il fasse fonctionner le dispositif de restriction.

41. Appareil selon la revendication 40, dans lequel le dispositif de fonctionnement comprend un système hydraulique et au moins une soupape pour commander un flux de fluide dans le système hydraulique.

42. Appareil selon la revendication 41, dans lequel le dispositif de commande comprend une commande à distance sans fil pour commander la soupape.

43. Appareil selon la revendication 40, dans lequel le dispositif de restriction comprend un système hydraulique et le dispositif de fonctionnement comprend un réservoir formant une chambre de fluide avec un volume variable connecté au système hydraulique, et le dispositif de fonctionnement est adapté pour distribuer du fluide depuis la chambre vers le système hydraulique par réduction du volume de la chambre et pour retirer du fluide du système hydraulique vers la chambre par expansion du volume de la chambre.

44. Appareil selon la revendication 38 ou 40, dans lequel le dispositif de fonctionnement comprend un moteur.

45. Appareil selon la revendication 44, dans lequel le moteur comprend un moteur rotatif, et le dispositif de commande commande le moteur rotatif, de façon à le faire tourner sur un certain nombre de tours.

46. Appareil selon la revendication 44, dans lequel le moteur comprend un moteur linéaire.

47. Appareil selon la revendication 44, dans lequel le moteur comprend un moteur fluide hydraulique ou pneumatique et le dispositif de commande commande le moteur de fluide.

48. Appareil selon la revendication 44, dans lequel le moteur comprend un moteur électrique ayant des parties électriquement conductrices faites de plastique.

49. Appareil selon l'une quelconque des revendications 1, 38 à 40, dans lequel le dispositif de commande libère l'énergie polarisée depuis la source d'énergie.

50. Appareil selon l'une quelconque des revendications 38 à 40, dans lequel le dispositif de commande change la polarité de l'énergie libérée pour inverser le dispositif de fonctionnement.

51. Appareil selon l'une quelconque des revendications 38 à 40, dans lequel le dispositif de fonctionnement comprend un moteur électrique et l'énergie libérée comprend l'énergie électrique.

52. Appareil selon l'une quelconque des revendications 1, 6, 38 à 40 et 44 comprenant en outre un dispositif d'inversion implantable dans le patient, afin d'inverser le dispositif de restriction.

53. Appareil selon la revendication 52, dans lequel le dispositif de commande commande le dispositif d'inversion pour inverser le dispositif de restriction.

54. Appareil selon la revendication 52, dans lequel le dispositif d'inversion comprend un système hydraulique, comprenant une soupape pour changer le sens du flux d'un fluide dans ledit système hydraulique.

55. Appareil selon la revendication 52, dans lequel le dispositif d'inversion comprend un dispositif d'inversion mécanique.

56. Appareil selon la revendication 55, dans lequel le dispositif d'inversion mécanique comprend un commutateur.

57. Appareil selon la revendication 55, dans lequel le dispositif d'inversion comprend une boîte d'engrenages.

58. Appareil selon la revendication 52, dans lequel le dispositif d'inversion comprend un commutateur.

59. Appareil selon la revendication 58, dans lequel le commutateur du dispositif d'inversion peut être actionné par l'énergie libérée.

60. Appareil selon la revendication 59, dans lequel le dispositif de commande commande le fonctionnement du commutateur du dispositif d'inversion en changeant la polarité de l'énergie libérée fournie au commutateur.

61. Appareil selon la revendication 58, dans lequel le commutateur comprend un commutateur électrique et la source d'énergie fournit de l'énergie électrique pour le fonctionnement du commutateur.

62. Appareil selon la revendication 58, dans lequel le dispositif de fonctionnement comprend un moteur, et le dispositif d'inversion inverse le moteur.

63. Appareil selon l'une quelconque des revendications 1, 2, 6, 38 à 40, dans lequel le dispositif de restriction comprend un système hydraulique et le dispositif de fonctionnement est adapté pour conduire un fluide hydraulique dans le système hydraulique.

64. Appareil selon la revendication 63, dans lequel le dispositif de fonctionnement comprend un moteur.

65. Appareil selon la revendication 63 ou 64, dans lequel le dispositif de fonctionnement comprend un conduit de fluide raccordé au système hydraulique du dispositif de restriction, et un réservoir pour le fluide, le réservoir faisant partie du conduit.

66. Appareil selon la revendication 65, dans lequel le système hydraulique et le conduit sont dénués de soupape anti-retour.

67. Appareil selon la revendication 66, dans lequel le réservoir forme une chambre de fluide avec un volume variable, et le dispositif de fonctionnement est adapté pour distribuer du fluide depuis la chambre vers le système hydraulique du dispositif de restriction en réduisant le volume de la chambre et pour retirer du fluide du système hydraulique vers la chambre par l'expansion du volume de la chambre.

68. Appareil selon l'une quelconque des revendications précédentes, comprenant en outre au moins un capteur implantable, destiné à détecter au moins un paramètre physique du patient.

69. Appareil selon la revendication 68, dans lequel le capteur comprend un capteur de pression pour détecter directement ou indirectement, en tant que paramètre physique, la pression dans le passage des aliments.

70. Appareil selon la revendication 68 ou 69, dans lequel le dispositif de commande commande le dispositif de restriction, en réponse aux signaux provenant du capteur.

71. Appareil selon la revendication 70, dans lequel le dispositif de commande comprend une unité de commande interne implantable dans le patient, l'unité de commande interne commandant le dispositif de restriction, en réponse aux signaux provenant du capteur.

72. Appareil selon la revendication 71, dans lequel le dispositif de commande comprend une unité de commande externe, située hors du corps du patient, l'unité de commande externe commandant le dispositif de restriction en réponse aux signaux provenant du capteur.

73. Appareil selon la revendication 72, dans lequel l'unité de commande externe mémorise l'information sur le paramètre physique détecté par le capteur et est manuellement actionnée pour commander le dispositif de restriction, sur la base des informations mémorisées.

74. Appareil selon l'une quelconque des revendications 68 à 73, comprenant en outre au moins un émetteur implantable pour envoyer les informations sur le paramètre physique détecté par le capteur.

75. Appareil selon l'une quelconque des revendications précédentes, comprenant en outre un communicateur de données externe, destiné à se trouver à l'extérieur du corps du patient, et un communicateur de données interne implantable dans le patient, afin de communiquer avec le communicateur externe, dans lequel le communicateur de données interne fournit des données concernant le patient au communicateur de données externe ou le communicateur de données externe fournit des données au communicateur de données interne.

76. Appareil selon la revendication 75, dans lequel le communicateur de données interne fournit des données concernant le dispositif de restriction.

77. Appareil selon la revendication 75 ou 76, dans lequel le communicateur implantable fournit des données concernant au moins un signal physique du patient.

78. Appareil selon l'une quelconque des revendications précédentes, dans lequel le dispositif de restriction est adapté pour commander la superficie transversale du passage d'aliments.

79. Appareil selon la revendication 78, dans lequel le dispositif de restriction peut être actionné pour ouvrir et fermer le passage d'aliments quand il est implanté dans le patient.

80. Appareil selon la revendication 78 ou 79, dans lequel le dispositif de restriction est adapté pour commander en continu la superficie transversale du passage d'aliments, quand il est implanté dans le patient.

81. Appareil selon l'une quelconque des revendications précédentes, comprenant en outre un dispositif de transformation de l'énergie implantable, dans lequel le dispositif de commande est adapté pour commander la source d'énergie, afin de libérer une énergie électrique sans fil et le dispositif de transformation d'énergie est adapté pour transformer l'énergie électrique en énergie cinétique pour le fonctionnement du dispositif de restriction.

82. Appareil selon la revendication 81, dans lequel le dispositif de restriction est directement actionné par l'énergie cinétique, quand le dispositif de transformation d'énergie transforme l'énergie électrique en énergie cinétique.

83. Appareil selon l'une quelconque des revendications précédentes, dans lequel le dispositif de restriction n'est pas gonflable.

84. Appareil selon l'une quelconque des revendications précédentes, dans lequel le dispositif de commande commande la source d'énergie pour libérer l'énergie pendant un laps de temps déterminé.

85. Appareil selon l'une quelconque des revendications 1 à 83, dans lequel le dispositif de commande commande la source d'énergie, afin de libérer l'énergie dans un nombre déterminé d'impulsions d'énergie.

86. Appareil selon l'une quelconque des revendications précédentes, dans lequel le dispositif de commande est adapté pour commander la source d'énergie, afin de libérer l'énergie de manière non invasive.

87. Appareil selon l'une quelconque des revendications précédentes, dans lequel le dispositif de commande comprend une commande à distance sans fil, pour transmettre au moins un signal de commande sans fil, afin de commander le dispositif de restriction.

88. Appareil selon la revendication 87, dans lequel la commande à distance est capable d'obtenir des informations sur la condition du dispositif de restriction, quand le dispositif de restriction est implanté et de commander le dispositif de restriction en réponse aux informations.

89. Appareil selon la revendication 87 ou 88, dans lequel la commande à distance sans fil comprend au moins un émetteur de signal ou émetteur-récepteur externe et au moins un récepteur de signal ou émetteur-récepteur interne implantable dans le patient.

90. Appareil selon la revendication 87 ou 88, dans lequel la commande à distance sans fil comprend au moins un récepteur de signal ou émetteur-récepteur externe et au moins un émetteur de signal ou émetteur-récepteur interne implantable dans le patient.

91. Appareil selon l'une quelconque des revendications 87 à 90, dans lequel la commande à distance est capable d'envoyer des informations concernant le dispositif de restriction depuis l'intérieur du corps du patient vers l'extérieur de celui-ci.

92. Appareil selon la revendication 91, dans lequel la commande à distance commande le dispositif de restriction, en réponse à l'information.

93. Appareil selon l'une quelconque des revendications 87 à 92, dans lequel la commande à distance transmet un signal de porteuse pour porter le signal de commande.

94. Appareil selon la revendication 93, dans lequel le signal de porteuse est modulé en fréquence ou en amplitude ou modulé en fréquence et en amplitude.

95. Appareil selon la revendication 93 ou 94, dans lequel le signal de porteuse est numérique, analogique ou numérique et analogique.

96. Appareil selon l'une quelconque des revendications 93 à 95, dans lequel le signal de commande utilisé avec le signal de porteuse est modulé en fréquence, en amplitude ou en fréquence et en amplitude.

97. Appareil selon la revendication 87 à 96, dans lequel le signal de commande comprend un champ électrique, magnétique ou électrique et magnétique.

98. Appareil selon l'une quelconque des revendications 87 à 96, dans lequel le signal de commande est numérique, analogique ou numérique et analogique.

99. Appareil selon la revendication 98, dans lequel la commande à distance transmet un signal d'onde porteuse électromagnétique pour transporter le signal de commande numérique ou analogique.

100. Appareil selon l'une quelconque des revendications 87 à 99, dans lequel le signal de commande est transmis par impulsions par la commande à distance sans fil.

101. Appareil selon l'une quelconque des revendications 2, 15 à 17, 79 à 81, comprenant en outre un stabilisateur implantable pour stabiliser l'énergie libérée par le dispositif de commande.

102. Appareil selon la revendication 101, dans lequel l'énergie libérée par le dispositif de commande comprend l'énergie électrique et le stabilisateur comprend au moins un condensateur.

103. Appareil selon la revendication 15 ou 16, dans lequel le dispositif de commande est adapté pour commander la source d'énergie, afin de libérer l'énergie électrique, et comprenant en outre un condensateur implantable pour produire le train d'impulsions énergétiques provenant de l'énergie libérée.

104. Appareil selon la revendication 1, comprenant en outre un dispositif de réglage pour régler le dispositif de restriction, afin de changer la taille de l'ouverture de l'estomac, dans lequel le dispositif de réglage est adapté pour régler mécaniquement le dispositif de restriction, ou adapté pour ajuster hydraulique-ment le dispositif de restriction, en utilisant un système hydraulique dénué de fluide hydraulique du type ayant une viscosité qui augmente sensiblement lorsqu'il est exposé à la chaleur ou à un champ magnétique.

105. Appareil selon la revendication 38, dans lequel le dispositif de fonctionnement comprend un dispositif de fonctionnement électrique.

106. Appareil selon la revendication 38, dans lequel le dispositif de fonctionnement est alimenté par l'énergie magnétique, l'énergie non magnétique, l'énergie électromagnétique, l'énergie non-électromagnétique, l'énergie cinétique, l'énergie non cinétique, l'énergie thermique ou l'énergie non-thermique.

107. Appareil selon la revendication 1, comprenant en outre des composants électriques implantables comprenant au moins une protection du niveau de tension.

108. Appareil selon la revendication 1, comprenant en outre des composants électriques implantables comprenant une protection de niveau de tension unique.

109. Appareil selon la revendication 107 ou 108, dans lequel les composants électriques sont dénués de détecteur de courant et/ou de détecteur de niveau de charge.

110. Appareil selon l'une quelconque des revendications 107 à 109, comprenant en outre un condensateur ou accumulateur implantable, dans lequel la charge ou la décharge du condensateur ou de l'accumulateur est commandé par l'utilisateur de la protection de niveau de tension.

111. Appareil selon la revendication 15, dans lequel l'énergie libérée comprend l'énergie électrique et comprenant en outre un condensateur implantable pour produire le train d'impulsions énergétiques.

112. Appareil selon la revendication 110 ou 111, dans lequel le condensateur a une capacité inférieure à 0,1 pF.

113. Appareil selon l'une quelconque des revendications 1, 2, 15 et 16, comprenant en outre un moteur ou une pompe implantable pour actionner le dispositif de restriction, dans lequel le dispositif de commande est adapté pour commander la source d'énergie, afin d'alimenter directement le moteur ou la pompe avec l'énergie libérée.

114. Appareil selon la revendication 15, dans lequel l'énergie sans fil comprend des ondes électromagnétiques à l'exclusion des ondes radio.

115. Appareil selon l'une quelconque des revendications 2, 15 et 16, comprenant en outre un moteur ou une pompe implantable pour actionner le dispositif de restriction, dans lequel le dispositif de commande est adapté pour libérer de l'énergie sans fil sous la forme d'un champ magnétique ou d'ondes électromagnétiques, pour alimenter directement le moteur ou la pompe, quand l'énergie sans fil est libérée.

116. Appareil selon la revendication 115, dans lequel la pompe n'est pas un type de pompe à piston.

117. Appareil selon l'une quelconque des revendications 2, 15 ou 16, dans lequel l'énergie sans fil comprend un signal.

118. Appareil selon la revendication 1, comprenant en outre un dispositif de transformation de l'énergie implantable, pour transformer l'énergie sans fil directement ou indirectement en énergie différente de l'énergie sans fil, afin de faire fonctionner le dispositif de restriction.

119. Appareil selon la revendication 118, dans lequel le dispositif de transformation d'énergie transforme l'énergie sans fil sous la forme d'ondes sonores en énergie électrique, pour faire fonctionner le dispositif de restriction.

120. Appareil selon la revendication 119, dans lequel le dispositif de transformation d'énergie transforme l'énergie sans fil sous la forme d'ondes sonores directement en énergie électrique.

121. Appareil selon la revendication 119 ou 120, dans lequel le dispositif de transformation d'énergie comprend un condensateur.

122. Appareil selon la revendication 121, dans lequel le condensateur est adapté pour produire des impulsions électriques provenant de l'énergie électrique transformée.

123. Appareil selon l'une quelconque des revendications 118 à 122, comprenant en outre un moteur ou une pompe implantable, pour faire fonctionner le dispositif de restriction, dans lequel le moteur ou la pompe est alimenté(e) par l'énergie transformée.

124. Appareil selon la revendication 1, dans lequel le dispositif de restriction est réglable de manière non manuelle.

125. Appareil selon l'une quelconque des revendications précédentes, dans lequel le dispositif de restriction est intégré dans un matériau semblable à un gel ou mou.

126. Appareil selon l'une quelconque des revendications précédentes, dans lequel le dispositif de restriction est intégré dans un matériau de silicone ayant une dureté inférieure à 20 Shore.
